# EUROPEAN PATENT APPLICATION

(11) **EP 4 807 014 A1**
(43) Date of publication of application: **16.09.2026**
(21) Application number: 26162816.8
(22) Date of filing: 06.03.2026
(51) Int. Cl.: C12P 17/18, C12N 9/02, C12N 9/10, C12N 15/70, C12N 15/81

(54) **ENZYMES FOR SYNTHESIZING GLABRIDIN**

(30) Priority: 11.03.2025 CN 202510280592
(71) Applicant: Tsinghua University, Beijing 100084 (CN)
(72) Inventor: Li, Chun, Beijing 100084 (CN); Zhang, Zhen, Beijing 100084 (CN); Qin, Lei, Beijing 100084 (CN)
(74) Representative: Petraz, Gilberto Luigi

(57) **Abstract**

Provided is a series of enzyme for synthesizing glabridin, a method for biosynthesizing glabridin, and an engineered biological host. The series of enzymes for synthesizing glabridin derived from *Glycyrrhiza glabra,* including a 4'-O-methyltransferase, an isoflavone 2'-hydroxylase, an isoflavone reductase, a vestitone reductase, a prenyltransferase, a pterocarpan reductase, an oxidative cyclase, and a demethylase, which are first disclosed in the present disclosure, exhibit respective activities in various biological hosts. In addition, for the first time, the heterologous biosynthesis of glabridin is realized, with a yield of glabridin reaching over 60%.

## Description

### FIELD

The present disclosure relates to the technical field of bioengineering and technology, in particular to an enzyme for synthesizing glabridin, a method for biosynthesizing glabridin, and an engineered biological host.

### BACKGROUND

Glabridin is a flavonoid compound extracted from *Glycyrrhiza glabra* L, having various physiological activities, such as anti-inflammatory property, antioxidant property, antibacterial property, tyrosinase inhibitory activity and the like. Therefore, glabridin is widely used in the field of brightening and skincare, for example, often as an ingredient in various cosmetics. In addition, glabridin has shown anti-cancer activity in a certain extent as well as potential values of application in aspects of anti-allergy, anti-virus, and cardiovascular system protection. Due to the natural origin and multiple pharmacological properties of the glabridin, it has gradually become a research hotspot in the fields of drug development, medical aesthetics, and functional foods.

At present, the glabridin is extracted mainly by the separation and purification from the roots of the plant *Glycyrrhiza glabra* (*Glycyrrhiza glabra L*.). This extraction method, however, is fraught with limitations stemming from multiple factors. For one, the glabridin content in *Glycyrrhiza glabra* is low, accounting for only approximately 0.09% of the plant's composition. What's more, the extraction process entails multiple steps including drying, grinding and comminution, often coupled with auxiliary extraction techniques such as ultrasonic and microwave treatment, which result in a cumbersome workflow, low extraction efficiency, high costs and significant environmental pollution. In addition, *Glycyrrhiza* features a well-developed root system with strong windbreak and sand-fixation capabilities. It is mainly distributed in Central Asia and northwest China, and in China, distributed in western regions such as Gansu, Xinjiang and Inner Mongolia with desert and semi-desert regions, endowing it with an ecological value. However, the above-mentioned uprooting extraction method purposes severely damages *Glycyrrhiza* vegetation and the local ecological environment. The *Glycyrrhiza* used at present is mostly derived from *Glycyrrhiza* imported and artificially cultivated. However, artificially cultivated *Glycyrrhiza* has drawbacks like a long growth cycle, occupation of arable land and high susceptibility to climatic conditions, all of which severely constrain glabridin production.

Therefore, there is an urgent need for an efficient and environmentally friendly method for synthesizing glabridin in the related art.

### SUMMARY

The present disclosure aims to solve at least one of the technical problems in the related art to a certain extent.

Therefore, in a first aspect, embodiments of the present disclosure provide a method for biosynthesizing glabridin, including:
providing a biological host;
engineering the biological host to heterologously express enzymes of a 4'-O-methyltransferase, an isoflavone 2'-hydroxylase, an isoflavone reductase, a vestitone reductase, a prenyltransferase, a pterocarpan reductase, an oxidative cyclase, and a demethylase to obtain an engineered biological host; and
culturing the engineered biological host to obtain the glabridin,
wherein the biological host is yeast,
wherein the glabridin is biosynthesized using daidzein as a substrate.

In some embodiments, wherein the enzymes heterologously expressed in the engineered biological host consist of the 4'-O-methyltransferase with an amino acid sequence of SEQ ID NO: 1 or SEQ ID NO: 2, the isoflavone 2'-hydroxylase with an amino acid sequence of SEQ ID NO: 3 or SEQ ID NO: 4, the isoflavone reductase with an amino acid sequence of SEQ ID NO: 5 or SEQ ID NO: 6, the vestitone reductase with an amino acid sequence of SEQ ID NO: 7 or SEQ ID NO: 8, the prenyltransferase with an amino acid sequence of SEQ ID NO: 16 or SEQ ID NO: 10, the pterocarpan reductase with an amino acid sequence of SEQ ID NO: 11 or SEQ ID NO: 12, the oxidative cyclase with an amino acid sequence of SEQ ID NO: 13, and the demethylase with an amino acid sequence of SEQ ID NO: 14 or SEQ ID NO: 15.

In some embodiments, wherein the engineered biological host heterologously expresses the 4'-O-methyltransferase with an amino acid sequence of SEQ ID NO: 1, the isoflavone 2'-hydroxylase with an amino acid sequence of SEQ ID NO: 3, the isoflavone reductase with an amino acid sequence of SEQ ID NO: 5, the vestitone reductase with an amino acid sequence of SEQ ID NO: 7, the prenyltransferase with an amino acid sequence of SEQ ID NO: 16, the pterocarpan reductase with an amino acid sequence of SEQ ID NO: 11, the oxidative cyclase with an amino acid sequence of SEQ ID NO: 13, and the demethylase with an amino acid sequence of SEQ ID NO: 14.

In some embodiments, wherein the engineered biological host heterologously expresses the 4'-O-methyltransferase with an amino acid sequence of SEQ ID NO: 2, the isoflavone 2'-hydroxylase an the amino acid sequence of SEQ ID NO: 4, the isoflavone reductase with an amino acid sequence of SEQ ID NO: 6, the vestitone reductase with an amino acid sequence of SEQ ID NO: 8, the prenyltransferase with an amino acid sequence of SEQ ID NO: 10, the pterocarpan reductase with an amino acid sequence of SEQ ID NO: 12, the oxidative cyclase with an amino acid sequence of SEQ ID NO: 13, and the demethylase with an amino acid sequence of SEQ ID NO: 15.

In some embodiments, wherein the yeast includes *Saccharomyces cerevisiae, Yarrowia lipolytica,* and *Pichia pastoris.*

Compared with the related art, the embodiments of the present disclosure achieve at least the following beneficial effects:
The present disclosure identifies a series of enzymes associated with glabridin biosynthesis from the plant *Glycyrrhiza glabra,* and enables the de novo biosynthesis of glabridin by heterologously expressing the identified enzymes in vitro, which include O-methyltransferase, isoflavone 2'-hydroxylase, isoflavone reductase, vestitone reductase, prenyltransferase, pterocarpan reductase, oxidative cyclase, and demethylase. Particularly, with use of the highly efficient and specific methyltransferases, prenyltransferases, pterocarpan reductases, oxidative cyclases, and demethylases screened, the present disclosure realizes, for the first time, the heterologous biosynthesis of glabridin from daidzein as raw material via biocatalysis, with the glabridin yield reaching over 60%.

The complete pathway and method for biosynthesizing glabridin according to the embodiments of the present disclosure are simple, efficient, and environmentally friendly, making it a promising approach for the large-scale industrial production of glabridin. This approach is expected to replace the existing plant extraction method for glabridin production, which relies on uprooting the plant and suffers from long production cycles, low efficiency, and prolonged occupation of arable land.

### DETAILED DESCRIPTION

The embodiments of the present disclosure are described below in combination with the detailed description in detail, and the embodiments given are illustrative only, not limiting the scope of the present disclosure. The embodiments provided below may serve as a guide for those skilled in the art to make further improvements, but do not constitute a limitation of the present disclosure in any way.

The present disclosure is made based on the following understanding of the inventors:
The related method, for extracting glabridin from the plant by uprooting, is inherently unsustainable and runs counter to the development of a green, eco-friendly ecological environment, for its long production cycles, low efficiency and prolonged occupation of arable land. Therefore, it is necessary to develop an innovative method that alter raw material sources to address these issues. Chemical synthesis methods, on the other hand, suffer from drawbacks including low yields, the requirement for product chiral resolution, harsh reaction conditions and complex operations. In the related art, research for the biosynthesis of glabridin has been limited to its precursor compounds such as 4'-O-methylpreglabridin, medicarpin and the like.

The inventors of the present disclosure identify a series of enzymes associated with glabridin biosynthesis from the plant *Glycyrrhiza glabra.* The present disclosure not only achieves the in-vitro biosynthesis of glabridin for the first time, but also enables the biosynthesis of glabridin using daidzein as a substrate, by expressing the identified enzymes in vitro, which include O-methyltransferase, isoflavone 2'-hydroxylase, isoflavone reductase, vestitone reductase, prenyltransferase, pterocarpan reductase, oxidative cyclase, and demethylase. Particularly, with use of the highly efficient and specific methyltransferases, prenyltransferases, pterocarpan reductases, oxidative cyclases, and demethylases screened, the present disclosure realizes, for the first time, the heterologous biosynthesis of glabridin from daidzein as raw material via biocatalysis, with the glabridin yield reaching over 60%.

The complete pathway and method for biosynthesizing glabridin according to the embodiments of the present disclosure are simple, efficient, and environmentally friendly, making it a promising approach for the large-scale industrial production of glabridin. This approach is expected to replace the existing plant extraction method for glabridin production, which relies on uprooting the plant and suffers from long production cycles, low efficiency, and prolonged occupation of arable land.

In a first aspect, embodiments of the present disclosure provide an enzyme for synthesizing glabridin, the enzyme includes one or more enzymes selected from:
a 4'-O-methyltransferase with an amino acid sequence depicted as SEQ ID NO: 2;
an isoflavone 2'-hydroxylase with an amino acid sequence depicted as SEQ ID NO: 4;
an isoflavone reductase with an amino acid sequence depicted as SEQ ID NO: 6;
a vestitone reductase with an amino acid sequence depicted as SEQ ID NO: 8;
a prenyltransferase with an amino acid sequence depicted as SEQ ID NO: 10;
a pterocarpan reductase with an amino acid sequence depicted as SEQ ID NO: 11 or 12;
an oxidative cyclase with an amino acid sequence depicted as SEQ ID NO: 13;
a demethylase with an amino acid sequence depicted as SEQ ID NO: 15.

In a second aspect, embodiments of the present disclosure provide a method for biosynthesizing glabridin, including: providing a biological host; enabling the biological host to express one or more enzymes selected from the group consisting of a 4'-O-methyltransferase, an isoflavone 2'-hydroxylase, an isoflavone reductase, a vestitone reductase, a prenyltransferase, a pterocarpan reductase, an oxidative cyclase, and a demethylase to obtain an engineered biological host; and culturing the engineered biological host to obtain the glabridin.

In some embodiments, wherein an amino acid sequence of the 4'-O-methyltransferase is selected from one or more of SEQ ID NO: 1 and SEQ ID NO: 2.

In some embodiments, an amino acid sequence of the isoflavone 2'-hydroxylase is selected from one or more of SEQ ID NO: 3 and SEQ ID NO: 4.

In some embodiments, an amino acid sequence of the isoflavone reductase is selected from one or more of SEQ ID NO: 5 and SEQ ID NO: 6.

In some embodiments, an amino acid sequence of the vestitone reductase is selected from one or more of SEQ ID NO: 7 and SEQ ID NO: 8.

In some embodiments, an amino acid sequence of the prenyltransferase is selected from one or more of SEQ ID NO: 9, SEQ ID NO: 10, and SEQ ID NO: 16.

In some embodiments, an amino acid sequence of the pterocarpan reductase is selected from one or more of SEQ ID NO: 11 and SEQ ID NO: 12.

In some embodiments, an amino acid sequence of the oxidative cyclase is selected from SEQ ID NO: 13.

In some embodiments, an amino acid sequence of the demethylase is selected from one or more of SEQ ID NO: 14 and SEQ ID NO: 15.

In some embodiments, wherein an amino acid sequence of the 4'-O-methyltransferase is depicted as SEQ ID NO: 2.

In some embodiments, an amino acid sequence of the isoflavone 2'-hydroxylase is depicted as SEQ ID NO: 4.

In some embodiments, an amino acid sequence of the isoflavone reductase is depicted as SEQ ID NO: 6.

In some embodiments, an amino acid sequence of the vestitone reductase is depicted as SEQ ID NO: 8.

In some embodiments, an amino acid sequence of the prenyltransferase is depicted as SEQ ID NO: 10.

In some embodiments, an amino acid sequence of the pterocarpan reductase is depicted as SEQ ID NO: 11 or SEQ ID NO: 12.

In some embodiments, an amino acid sequence of the oxidative cyclase is depicted as SEQ ID NO: 13.

In some embodiments, an amino acid sequence of the demethylase is depicted as SEQ ID NO: 15.

In some embodiments, wherein the biological host is selected from the group consisting of a gram-positive bacterium, a gram-negative bacterium, an actinomycete, a yeast, a filamentous fungus, and a *Nicotiana* species.

In some embodiments, the dram-positive bacterium includes *Bacillus subtilis* and *Corynebacterium glutamicum,* the gram-negative bacterium includes *Escherichia coli,* the actinomycete includes *Streptomyces coelicolour,* the yeast includes *Saccharomyces cerevisiae, Yarrowia lipolytica,* and *Pichia pastoris,* the filamentous fungus includes *Aspergillus niger,* and the *Nicotiana* species comprises *Nicotiana benthamiana.*

In some embodiments, wherein the glabridin is biosynthesized using a substrate selected from the group consisting of daidzein, formononetin, vestitone, and medicarpin.

In some embodiments, wherein the biological host is *Nicotiana benthamiana,* wherein the engineered biological host heterologously expresses the prenyltransferase with an amino acid sequence depicted as SEQ ID NO: 10, the pterocarpan reductase with an amino acid sequence depicted as SEQ ID NO: 11, the oxidative cyclase with an amino acid sequence depicted as SEQ ID NO: 13, and the demethylase with an amino acid sequence depicted as SEQ ID NO: 15, and wherein a substrate for biosynthesizing the glabridin is medicarpin.

In some embodiments, wherein the biological host is yeast, wherein the engineered biological host heterologously expresses the 4'-O-methyltransferase with an amino acid sequence shown in SEQ ID NO: 1, the isoflavone 2'-hydroxylase with an amino acid sequence shown in SEQ ID NO: 3, the isoflavone reductase with an amino acid sequence depicted as SEQ ID NO: 5, the vestitone reductase with an amino acid sequence depicted as SEQ ID NO: 7, the prenyltransferase with an amino acid sequence depicted as SEQ ID NO: 16, the pterocarpan reductase with an amino acid sequence depicted as SEQ ID NO: 11, the oxidative cyclase with an amino acid sequence depicted as SEQ ID NO: 13, and the demethylase with an amino acid sequence depicted as SEQ ID NO: 14, and wherein a substrate for biosynthesizing the glabridin is daidzein.

In some embodiments, wherein the biological host is yeast, wherein the engineered biological host heterologously expresses the 4'-O-methyltransferase with an amino acid sequence depicted as SEQ ID NO: 2, the isoflavone 2'-hydroxylase an the amino acid sequence depicted as SEQ ID NO: 4, the isoflavone reductase with an amino acid sequence depicted as SEQ ID NO: 6, the vestitone reductase with an amino acid sequence depicted as SEQ ID NO: 8, the prenyltransferase with an amino acid sequence depicted as SEQ ID NO: 10, the pterocarpan reductase with an amino acid sequence depicted as SEQ ID NO: 12, the oxidative cyclase with an amino acid sequence depicted as SEQ ID NO: 13, and the demethylase with an amino acid sequence depicted as SEQ ID NO: 15, and wherein a substrate for biosynthesizing the glabridin is daidzein.

In a third aspect, embodiments of the present disclosure provide an engineered biological host, expressing one or more enzymes selected from the group consisting of:
a 4'-O-methyltransferase with an amino acid sequence depicted as SEQ ID NO: 2;
an isoflavone 2'-hydroxylase with an amino acid sequence depicted as SEQ ID NO: 4;
an isoflavone reductase with an amino acid sequence depicted as SEQ ID NO: 6;
a vestitone reductase with an amino acid sequence depicted as SEQ ID NO: 8;
a prenyltransferase with an amino acid sequence depicted as SEQ ID NO: 10;
a pterocarpan reductase with an amino acid sequence depicted as SEQ ID NO: 11 or SEQ ID NO: 12;
an oxidative cyclase with an amino acid sequence depicted as SEQ ID NO: 13;
a demethylase with an amino acid sequence depicted as SEQ ID NO: 15.

In a fourth aspect, embodiments of the present disclosure provide use of a 4'-O-methyltransferase with an amino acid sequence depicted as SEQ ID NO: 1 in synthesizing formononetin or glabridin.

In a fifth aspect, embodiments of the present disclosure provide use of an isoflavone 2'-hydroxylase with an amino acid sequence depicted as SEQ ID NO: 3 in synthesizing 2'-hydroxyformononetin or glabridin.

In a sixth aspect, embodiments of the present disclosure provide use of an isoflavone reductase with an amino acid sequence depicted as SEQ ID NO: 5 in synthesizing vestitone or glabridin.

In a seventh aspect, embodiments of the present disclosure provide use of a vestitone reductase with an amino acid sequence depicted as SEQ ID NO: 7 in synthesizing medicarpin or glabridin.

In an eighth aspect, embodiments of the present disclosure provide use of a prenyltransferase with an amino acid sequence depicted as SEQ ID NO: 9 or SEQ ID NO: 16 in synthesizing glabridin.

In a nineth aspect, embodiments of the present disclosure provide use of a demethylase with an amino acid sequence depicted as SEQ ID NO: 14 in synthesizing glabridin.

It can be understood that the uses according to the embodiments of the fourth to ninth aspects of the present disclosure include, but are not limited to, intracellular catalysis, extracellular catalysis, enzymatic catalysis, or cell-free catalysis.

The biological hosts applicable to the embodiments of the second aspect are identical to those for the uses set forth in the fourth to ninth aspects of the present disclosure, and will not be repeated here.

### Terms

As used herein, the terms "engineered strain", "engineered plant", and "engineered biological host" refer to a bacterial or fungal cell line or a plant that efficiently expresses exogenous genes via genetic engineering techniques.

As used herein, the terms "starting strain", "chassis strain", and "biological host" refer to an original strain of bacterium or fungus or an original plant used for genetic engineering.

As used herein, the term "genetic circuit" refers to a gene expression system containing all essential elements required for the expression of a target polypeptide, typically including the following elements: a promoter, an encoding reporter gene, and a terminator.

As used herein, the term "comprising" or "including" is an open-ended expression. When the term is used to describe a sequence of a protein or a nucleic acid, the protein or the nucleic acid may consist of the recited sequence alone, or may have additional amino acid or nucleotide residues at one or both ends, while still retaining the same or similar biological activity as the original sequence.

As used herein, the term "comprising" or "including" is an open-ended description that encompasses the specified components or steps, as well as other components or steps that do not substantially influence the disclosed technical solution.

As used herein, the term "and/or" includes all possible combinations of the items it connects, with each combination deemed to be individually recited herein. For example, "A and/or B" includes "A", "A and B", and "B". In another example, "A, B and/or C" includes "A", "B", "C", "A and B", "A and C", "B and C", and "A, B and C".

The abbreviations used herein and their full names are provided in Table 1 below.

**Table 1**

| Abbreviation | Full Name |
|---|---|
| GgOMT | 4'-O-methyltransferase derived from *Glycyrrhiza glabra* |
| PlOMT9 | 4'-O-methyltransferase derived from *Pueraria lobata* |
| MES | 2-(N-morpholino)ethanesulfonic acid |
| AS | Acetosyringone |
| GgI2'H | Isoflavone 2'-hydroxylase derived from *Glycyrrhiza glabra* |
| GeCYP81E1 | Isoflavone 2'-hydroxylase derived from *Glycyrrhiza echinata* |
| IPTG | Isopropyl-β-D-thiogalactopyranoside |
| GgIFR | Isoflavone reductase derived from *Glycyrrhiza glabra* |
| MsIFR | Isoflavone reductase derived from *Medicago sativa* |
| GgVR | Vestitone reductase derived from *Glycyrrhiza glabra* |
| MsVR | Vestitone reductase derived from *Medicago sativa* |
| GgPT | Prenyltransferase derived from *Glycyrrhiza glabra* |
| PcM4DT/tPcM4DT | *pterocarpan prenyltransferase* derived from *Cullen corylifolium* |
| GgPTR | Pterocarpan reductase derived from *Glycyrrhiza glabra* |
| GgOC | Oxidative cyclase derived from *Glycyrrhiza glabra* |
| GgDMT | Demethylase derived from *Glycyrrhiza glabra* |
| NhDMT | Demethylase derived from *Nectria haematococca* |

### Sources of Experimental Materials

Wild-type (WT) *Nicotiana benthamiana* is described in the article "Yuan J, Yu Z, Li Y, Shah SHA, Xiao D, Hou X, Li Y. Ectopic expression of BrIQD35 promotes drought stress tolerance in Nicotiana benthamiana. Plant Biol (Stuttg). 2022 Aug;24(5):887-896." The material is available to the public from the applicant for the purpose of repeating the experiments of the present disclosure only.

*Streptomyces coelicolor* (*S. coelicolor*) A3(2) is described in the article "Hopwood DA (1999) Forty years of genetics with Streptomyces: from in vivo through in vitro to in silico. Microbiology 145(9):2183-2202." The material is available to the public from the applicant for the purpose of repeating the experiments of the present disclosure only.

*Yarrowia lipolytica* ATCC 201249 is described in the article "Du H-X, Xiao W-H, Wang Y, Zhou X, Zhang Y, Liu D, et al. (2016) Engineering Yarrowia lipolytica for Campesterol Overproduction. PLoS ONE 11(1): e0146773." The material is available to the public from the applicant for the purpose of repeating the experiments of the present disclosure only.

The present disclosure is further illustrated with the specific examples of glabridin heterologous biosynthesis below. The specific examples are provided only for explanatory purposes and are not intended to limit the scope of the present disclosure. Without departing from the scope of the appended claims, those skilled in the art may make various modifications to the aspects of the present disclosure, and all such modifications shall also fall within the protection scope of the present disclosure. For example, those skilled in the art may understand and carry out the replacement of the cellular expression systems and vector plasmids used in the present examples with other conventional expression systems and vectors in the art.

The materials, reagents, etc. used in the following examples are commercially available unless otherwise specified.

### Example 1: Expression and Functional Verification of 4'-O-methyltransferase

4'-O-methyltransferase derived from *Glycyrrhiza glabra* (GgOMT), with the amino acid sequence of SEQ ID NO: 2 and the nucleotide sequence of SEQ ID NO: 17 accordingly, was selected and subjected to gene synthesis according to the sequence provided. The synthesized gene was amplified by PCR and ligated into the *Agrobacterium* expression vector pBI121 using Gibson assembly to obtain a recombinant plasmid. The recombinant plasmid was introduced into *Agrobacterium* competent cells EHA105 by electroporation. *Agrobacterium* clones transformed with the expression plasmid successfully were picked and cultured in 1 ml of LB medium containing the corresponding antibiotics at 28 °C with shaking at 250 rpm for 24 hours. Then, 50 µl of cultured *Agrobacterium* solution was inoculated into 5 ml of LB medium containing the corresponding antibiotics as well as with 100 µl of 0.5 M MES and 2 µl of 100 mM AS added, and then further cultured at 28 °C with shaking at 250 rpm until OD600=1.0. A centrifugation at 4000 rpm for 10 minutes was performed, and the precipitate of bacterial cells was collected and resuspended in 10 mM MgCl₂ to OD600=1.0. Then, 2 µl of 100 mM AS was added per ml of bacterial resuspended solution, followed by standing for more than 3 hours.

*Nicotiana benthamiana* in the vigorous growth period was selected. The bacterial solution was drawn up into a syringe without a needle. With fingers pressing against the front of the leaf, the bacterial solution was infiltrated from the back of the leaf using the syringe. 1 mM daidzein solution as the reaction substrate was injected at the same time in the same way. The treated plant was kept for 24-48 hours, and then subjected to sample collection for product detection.

The collected leaves were cut into pieces and subjected to bead-beating disruption at -20 °C. After the disruption, the precipitate and the separated supernatant were extracted with an equal volume of ethyl acetate, and the obtained organic phase was evaporated to dryness and redissolved with methanol. The production of formononetin was detected by ultra-performance liquid chromatography (UPLC) or liquid chromatography-mass spectrometry (LC-MS).

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A consisted of 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of formononetin.

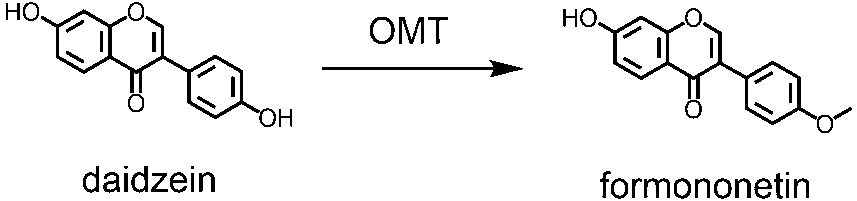

### Example 2: Expression and Functional Verification of Isoflavone 2'-hydroxylase

Isoflavone 2'-hydroxylase derived from *Glycyrrhiza glabra* (GgI2'H), with the amino acid sequence of SEQ ID NO: 4 and the nucleotide sequence of SEQ ID NO: 18 accordingly, was selected and subjected to gene synthesis according to the sequence provided. The synthesized gene was amplified by PCR and ligated into the *Escherichia coli* expression vector pET28a using Gibson assembly to obtain a recombinant plasmid. The recombinant plasmid was introduced into *Escherichia coli* competent cells BL21 by electroporation. *Escherichia coli* clones transformed with the expression plasmid successfully were picked and cultured in 1 mL of LB medium containing the corresponding antibiotics at 37 °C with shaking at 220 rpm for 12 hours. Then, 0.1 mL of cultured bacterial solution was inoculated into 5 mL of LB medium containing the corresponding antibiotics, and cultured until the OD600=0.6-0.8 as determined using a UV spectrophotometer. The temperature of the shaker was reduced to 16 °C, and 1 mM IPTG was added to the bacterial solution for induction. After 10 hours, 1 mM formononetin was added as the reaction substrate, the temperature of the shaker was adjusted to 30 °C, and samples were collected for product detection after 24-48 hours of reaction.

The reacted culture solution was centrifuged, and the supernatant was collected and extracted with an equal volume of ethyl acetate. The obtained organic phase was evaporated to dryness and redissolved with methanol. The production of 2'-hydroxyformononetin was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of 0.96 mM 2'-hydroxyformononetin.

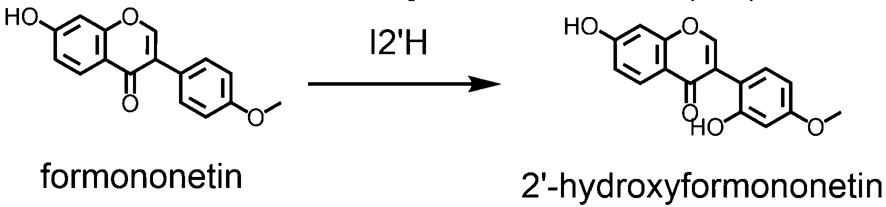

### Example 3: Expression and Functional Verification of Isoflavone Reductase

Isoflavone reductase derived from *Glycyrrhiza glabra* (GgIFR), with the amino acid sequence of SEQ ID NO: 6 and the nucleotide sequence of SEQ ID NO: 19 accordingly, was selected and subjected to gene synthesis according to the sequence provided. The synthesized gene was amplified by PCR and ligated into the *Saccharomyces cerevisiae* expression vector pYES2 using Gibson assembly to obtain a recombinant plasmid. The recombinant plasmid was introduced into *Escherichia coli* competent cells DH5α by electroporation. *Escherichia coli* clones transformed with the expression plasmid successfully were picked and cultured in 1 mL of LB medium containing the corresponding antibiotics at 37 °C with shaking at 220 rpm for 12 hours. The bacterial cells were collected by centrifugation to extract the plasmid. The plasmid was transformed into *Saccharomyces cerevisiae* INVSC cells. The positive clones obtained after transformation were inoculated into SD-Ura medium and cultured at 30 °C with shaking at 250 rpm for 16 hours. Then, 20 g/L galactose was added for induction, and 1 mM 2'-hydroxyformononetin was added as the reaction substrate at the same time. The reaction was carried out at 30 °C with shaking at 220 rpm for 24-48 hours, and samples were collected for product detection.

The reacted culture solution was centrifuged, and the supernatant was collected and extracted with an equal volume of ethyl acetate. The obtained organic phase was evaporated to dryness and redissolved with methanol. The production of vestitone was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of 0.88 mM vestitone.

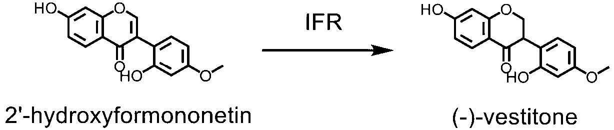

### Example 4: Expression and Functional Verification of Vestitone Reductase

Vestitone reductase derived from *Glycyrrhiza glabra* (GgVR), with the amino acid sequence of SEQ ID NO: 8 and the nucleotide sequence of SEQ ID NO: 20 accordingly, was selected and subjected to gene synthesis according to the sequence provided. The synthesized gene was amplified by PCR and ligated into the *Saccharomyces cerevisiae* expression vector pAK203 using Gibson assembly to obtain a recombinant plasmid. The recombinant plasmid was introduced into Escherichia coli competent cells DH5α by electroporation. *Escherichia coli* clones transformed with the expression plasmid successfully were picked and cultured in 1 mL of LB medium containing the corresponding antibiotics at 37 °C with shaking at 220 rpm for 12 hours. The bacterial cells were collected by centrifugation to extract the plasmid. The plasmid was transformed into *Streptomyces coelicolor* cells by protoplast transformation. The positive clones obtained after transformation were inoculated into medium and cultured at 30 °C with shaking at 250 rpm for 24 hours. Then, thiostrepton was added for induction, and 1 mM vestitone was added as the reaction substrate at the same time. The reaction was carried out at 30 °C with shaking at 220 rpm for 24-48 hours, and samples were collected for product detection.

The reacted culture solution was centrifuged, and the supernatant was collected and extracted with an equal volume of ethyl acetate. The obtained organic phase was evaporated to dryness and redissolved with methanol. The production of medicarpin was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of 0.9 mM medicarpin.

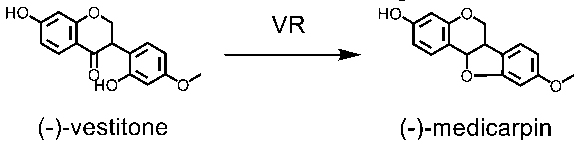

### Example 5: Expression and Functional Verification of Prenyltransferase, Pterocarpan Reductase, Oxidative Cyclase, and Demethylase

Prenyltransferase derived from *Glycyrrhiza glabra* (GgPT) with the amino acid sequence of SEQ ID NO: 10, pterocarpan reductase derived from *Glycyrrhiza glabra* (GgPTR) with the amino acid sequence of SEQ ID NO: 11, oxidative cyclase derived from *Glycyrrhiza glabra* (GgOC) with the amino acid sequence of SEQ ID NO: 13, and demethylase derived from *Glycyrrhiza glabra* (GgDMT) with the amino acid sequence of SEQ ID NO: 15 were selected and subjected to gene synthesis according to the sequences provided. Their corresponding nucleotide sequences are SEQ ID NO: 21, SEQ ID NO: 22, SEQ ID NO: 23, and SEQ ID NO: 24 in sequence. The synthesized genes were amplified by PCR and ligated into the *Agrobacterium* expression vector pBI121 using Gibson assembly to obtain a recombinant plasmid. The recombinant plasmid was introduced into *Agrobacterium* competent cells EHA105 by electroporation. *Agrobacterium* clones successfully transformed with the expression plasmid were picked and cultured in 1 mL of LB medium containing the corresponding antibiotics at 28 °C with shaking at 250 rpm for 24 hours. Then, 50 µl of cultured *Agrobacterium* solution was inoculated into 5 ml of LB medium containing the corresponding antibiotics as well as with 100 µl of 0.5 M MES and 2 µl of 100 mM AS added, and then further cultured at 28 °C with shaking at 250 rpm until OD600=1.0. A centrifugation at 4000 rpm for 10 minutes was performed, and the precipitate of bacterial cells was collected and resuspended in 10 mM MgCl₂ to OD600=1.0. Then, 2 µl of 100 mM AS was added per ml of bacterial resuspended solution, followed by standing for more than 3 hours.

*Nicotiana benthamiana* in the vigorous growth period was selected. The bacterial resuspension was drawn up into a syringe without a needle; with fingers pressing against the front of the leaf, the bacterial solution was infiltrated from the back of the leaf, and 1 mM medicarpin solution was injected as the reaction substrate at the same time. Samples were collected for product detection after 24-48 hours of placement.

The collected leaves were cut into pieces and subjected to bead-beating disruption at -20 °C. After disruption, the precipitate and the separated supernatant were extracted with an equal volume of ethyl acetate. The obtained organic phase was evaporated to dryness and redissolved with methanol. The production of glabridin was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of glabridin.

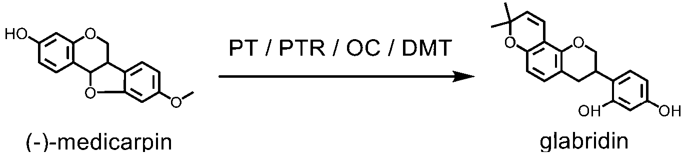

### Example 6: Construction and Verification of the Pathway for Synthesizing Glabridin Using Daidzein as Substrate

4'-O-methyltransferase derived from *Pueraria lobata* (PlOMT9) with the amino acid sequence of SEQ ID NO: 1, isoflavone 2'-hydroxylase derived from *Glycyrrhiza echinata* (GeCYP81E1) with the amino acid sequence of SEQ ID NO: 3, isoflavone reductase derived from *Medicago sativa* (MsIFR) with the amino acid sequence of SEQ ID NO: 5, vestitone reductase derived from *Medicago sativa* (MsVR) with the amino acid sequence of SEQ ID NO: 7, *pterocarpan prenyltransferase* derived from *Cullen corylifolium* (tPcM4DT) with the amino acid sequence of SEQ ID NO: 16, pterocarpan reductase derived from *Glycyrrhiza glabra* (GgPTR) with the amino acid sequence of SEQ ID NO: 11, oxidative cyclase derived from *Glycyrrhiza glabra* (GgOC) with the amino acid sequence of SEQ ID NO: 13, and demethylase derived from *Nectria haematococca* (NhDMT) with the amino acid sequence of SEQ ID NO: 14 were selected and subjected to gene synthesis according to the sequences provided. The corresponding nucleotide sequences of the enzymes are SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32 in sequence, respectively. The synthesized genes were amplified by PCR, ligated into an expression cassette using Overlap Extension-PCR (OE-PCR) and Gibson assembly, and constructed into the commonly used vector plasmid pYL15 (GenBank accession: KU378202) for *Yarrowia lipolytica.* The expression cassette sequence was: P_{TDH}-PlOMT9-T_{VMA}-P_{FBA}-GmCYP81E1-T_{CYC}-P_{TEF}-MsIFR-T_{CDC}-P_{TEF}-MsVR-T_{ATP}-P_{ENO}-tPcM4 DT-T_{ADH}-P_{TDH}-Gg*PTR*-T_{RPL}-P_{HSP12}-Gg*OC*-T_{SOD1}-P_{HSP26}-Nh*DMT*-T_{FBP}-URA3. The recombinant plasmid was introduced into *Escherichia coli* competent cells DH5α by transformation. *Escherichia coli* clones successfully transformed with the expression plasmid were picked and cultured in 1 mL of LB medium containing the corresponding antibiotics at 37 °C with shaking at 220 rpm for 12 hours. The bacterial cells were collected by centrifugation to extract the plasmid. The plasmid was transformed into *Yarrowia lipolytica* ATCC201249. The positive clones obtained after transformation were inoculated into SD-Ura medium and cultured at 30 °C with shaking at 250 rpm for 16 hours. Then, 5 mM daidzein was added as the reaction substrate, and the reaction was carried out at 30 °C with shaking at 220 rpm for 96 hours, then samples were collected for product detection.

The reacted culture was subjected to bead-beating disruption and extracted with an equal volume of ethyl acetate. The obtained organic phase was evaporated to dryness and redissolved with methanol. The production of glabridin was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of 1.8 mM glabridin.

### Example 7: Construction and Verification of the Pathway for Synthesizing Glabridin Using Daidzein as Substrate

4'-O-methyltransferase derived from *Glycyrrhiza glabra* (GgOMT) with the amino acid sequence of SEQ ID NO: 2, isoflavone 2'-hydroxylase derived from *Glycyrrhiza glabra* (GgI2'H) with the amino acid sequence of SEQ ID NO: 4, isoflavone reductase derived from *Glycyrrhiza glabra* (GgIFR) with the amino acid sequence of SEQ ID NO: 6, vestitone reductase derived from *Glycyrrhiza glabra* (GgVR) with the amino acid sequence of SEQ ID NO: 8, prenyltransferase derived from *Glycyrrhiza glabra* (GgPT) with the amino acid sequence of SEQ ID NO: 10, pterocarpan reductase derived from *Glycyrrhiza glabra* (GgPTR) with the amino acid sequence of SEQ ID NO: 12, oxidative cyclase derived from *Glycyrrhiza glabra* (GgOC) with the amino acid sequence of SEQ ID NO: 13, and demethylase derived from *Glycyrrhiza glabra* (GgDMT) with the amino acid sequence of SEQ ID NO: 15 were selected and subjected to gene synthesis according to the sequences provided. The corresponding nucleotide sequences of the enzymes are SEQ ID NO: 33, SEQ ID NO: 34, SEQ ID NO: 19, SEQ ID NO: 35, SEQ ID NO: 36, SEQ ID NO: 37, SEQ ID NO: 38, and SEQ ID NO: 39 in sequence, respectively. The synthesized genes were amplified by PCR, ligated into the expression cassette of HOL-P_{TDH3}-GgOMT-T_{VMA1}-P_{FBA1}-GgI2'H-T_{CYC1}-P_{TEF1}-GgIFR-T_{CDC19}-P_{TEF2}-GgVR-T_{ATP1}-P_{ENO2} -GgPT-T_{ADH1}-P_{TDH1}-Gg*PTR*-T_{RPL1}-P_{HSP12}-Gg*OC*-T_{SOD1}-P_{HSP26}-Gg*DMT*-T_{FBP1}-URA3-HOR using OE-PCR and Gibson assembly, and further ligated into the vector pUC19. The recombinant plasmid was introduced into *Escherichia coli* competent cells DH5α by transformation. *Escherichia coli* clones successfully transformed with the expression plasmid were picked and cultured in 1 mL of LB medium containing the corresponding antibiotics at 37 °Cwith shaking at 220 rpm for 12 hours. The bacterial cells were collected by centrifugation to extract the plasmid. DNA fragments ①HOL-P_{TDH3}-GgOMT-T_{VMA1}-P_{FBA1}-GgI2'H-T_{CYC1}-P_{TEF1}-GgIFR-T_{CDC19}, ②T_{CDC19}-P_{TEF2}-GgVR-T_{ATP1}-P_{ENO2}-GgPT-T_{ADH1}-P_{TDH1}-Gg*PTR*-T_{RPL1}-P_{HSP12}, ③P_{HSP12}-Gg*OC*-T_{SOD1}-P_{HSP26}-Gg*DMT*-T_{FBP1}-URA3-HOR were amplified by PCR, respectively, and then simultaneously transformed into *Saccharomyces cerevisiae* BY4741. The positive clones obtained after transformation were inoculated into SD-Ura medium and cultured at 30 °Cwith shaking at 250 rpm for 16 hours. Then, 1 mM daidzein was added as the reaction substrate, and the reaction was carried out at 30 °C with shaking at 220 rpm for 96 hours, then samples were collected for product detection.

The reacted culture was subjected to bead-beating disruption and extracted with an equal volume of ethyl acetate. The obtained organic phase was evaporated to dryness and redissolved with methanol. The production of glabridin was detected by UPLC or LC-MS.

Specifically, quantitative detection of the fermentation products from the engineered strains was performed using a UPLC instrument (Agilent 1260). Separation was carried out on a Poroshell 120 EC-C18 column of particle size 2.7 µm and 3.0 × 100 mm. The detection wavelength was set at 254 nm, and the column oven temperature was maintained at 35 °C. Mobile phase A was 0.1% formic acid in water, and mobile phase B was acetonitrile. Gradient elution was performed as follows: 0 min, 20% B; 0-4.5 min, 20%-35% B; 4.5-6 min, 35%-50% B; 6-12 min, 50%-65% B; 12-18 min, 65%-85% B; 18-22.5 min, 85%-95% B; 22.5-24 min, 95%-20% B; and 24-30 min, 20% B. The flow rate was 0.2 mL/min, and the injection volume was 1 µL. Qualitative and quantitative detection were performed using the external standard method. The detection results confirmed the production of 0.6 mM glabridin.

The enzymes, biological hosts, substrates, and products described in the examples above are shown in Table 3 below. It can be seen from Table 2 that the series of enzymes for synthesizing glabridin derived from *Glycyrrhiza glabra,* which are first disclosed in the present disclosure, exhibit respective activities in various biological hosts. In addition, for the first time, the heterologous biosynthesis of glabridin is realized, with a yield of glabridin reaching over 60%.

**Table 2**

| Example | Enzyme And Origin Thereof | Chassis Biological Host | Substrate | Product |
|---|---|---|---|---|
| Example 1 | 4'-O-methyltransferase derived from *Glycyrrhiza glabra* (GgOMT), SEQ ID NO: 2 | wild -type *Nicotiana benthamiana* | 1mM daidzein | 4.5 mg formonon etin/g *Nicotiana* species |
| Example 2 | Isoflavone 2'-hydroxylase derived from *Glycyrrhiza glabra* (GgI2'H), SEQ ID NO: 4 | *Escherichia coli* BL21 | 1mM formononetin | 0.96mM 2'-hydrox yformono netin |
| Example 3 | Isoflavone reductase derived from *Glycyrrhiza glabra* (GgIFR), SEQ ID NO: 6 | *Saccharomyces cerevisiae* INVSC1 | 1mM 2'-hydroxyf ormononetin | 0.88mM vestitone |
| Example 4 | Vestitone reductase derived from *Glycyrrhiza glabra* (GgVR), SEQ ID NO: 8 | *Streptomyces coelicolor* (*S. coelicolor*) A3(2) | 1mM vestitone | 0.9mM medicarpin |
| Example 5 | Prenyltransferase derived from *Glycyrrhiza glabra* (GgPT), SEQ ID NO: 10; | wild -type *Nicotiana benthamiana* | 1mM medicarpin | 7.3 mg glabridin /g *Nicotiana* species |
| | pterocarpan reductase derived from *Glycyrrhiza glabra* (GgPTR), SEQ ID NO: 11; | | | |
| | oxidative cyclase derived from *Glycyrrhiza glabra* (GgOC) , SEQ ID NO: 13; | | | |
| | demethylase derived from *Glycyrrhiza glabra* (GgDMT), SEQ ID NO: 15 | | | |
| Example 6 | 4'-O-methyltransferase derived from *Pueraria lobata* (PlOMT9) , SEQ ID NO: 1; | *Yarrowia lipolytica* ATCC 201249 | 5mM daidzein | 1.8mM glabridin |
| | isoflavone 2'-hydroxylase derived from *Glycyrrhiza echinata* (GeCYP81E1), SEQ ID NO: 3; | | | |
| | isoflavone reductase derived from *Medicago sativa* (MsIFR), SEQ ID NO: 5; | | | |
| | vestitone reductase derived from *Medicago sativa* (MsVR), SEQ ID NO: 7; | | | |
| | *pterocarpan prenyltransferase* derived from *Cullen corylifolium* (tPcM4DT) , SEQ ID NO: 16; | | | |
| | pterocarpan reductase derived from *Glycyrrhiza glabra* (GgPTR), SEQ ID NO: 11; | | | |
| | oxidative cyclase derived from *Glycyrrhiza glabra* (GgOC) ,SEQ ID NO: 13; | | | |
| | demethylase derived from *Nectria haematococca* (NhDMT), SEQ ID NO: 14 | | | |
| Example 7 | 4'-O-methyltransferase derived from *Glycyrrhiza glabra* (GgOMT) , SEQ ID NO: 2; | *Saccharomyces cerevisiae* BY4741 | 1mM daidzein | 0.6mM glabridin |
| | isoflavone 2'-hydroxylase derived from *Glycyrrhiza glabra* (GgI2'H) , SEQ ID NO: 4; | | | |
| | isoflavone reductase derived from *Glycyrrhiza glabra* (GgIFR) , SEQ ID NO: 6; | | | |
| | vestitone reductase derived from *Glycyrrhiza glabra* (GgVR) , SEQ ID NO: 8; | | | |
| | prenyltransferase derived from *Glycyrrhiza glabra* (GgPT), SEQ ID NO: 10; | | | |
| | pterocarpan reductase derived from *Glycyrrhiza glabra* (GgPTR) , SEQ ID NO: 12; | | | |
| | oxidative cyclase derived from *Glycyrrhiza glabra* (GgOC) , SEQ ID NO: 13; | | | |
| | demethylase derived from *Glycyrrhiza glabra* (GgDMT) , | | | |
| | SEQ ID NO: 15 | | | |

### Sequence Information

>SEQ ID NO: 1 (PlOMT9) 
MGSNNGRKASEIFQGQALLYRHMFAFVDSMCLKSIVELGIPNIIHKHGQPITLSELMS
ILQVPPAKVGHVQSLMRYLAHHGFFERLRIHEKDAYALTAASELLVKGTEPYLAPMVEC
MLDPTLSASFHQMKKWVYEEDLSVFDISLGSNLWDFLTKNPAYNEIFNEAMASDSQMSN
LALRDCKLVFEGLESIVDVGGGTGTTAKIICEAFPNLKCIVFDRPQVVENLLENNNLTYV
GGDMFKSIPKANAVLLKWILHDWTDKDCRKILENCKEAISNNSKRGKIIIIDIVINEKQDE
HKVTELKLLFDVAMACVLNGKERNEEEWKKLFMEVGLQDYKISPLTGYLSLIEIYP*
>SEQ ID NO: 2 (GgOMT) 
MDFSTNGSEESELYHAQIHLYKHVYNFVSSMALKSAMELGIADVIHNHGKPITLPQL
ASALKLHPSKVGVLYRFLRLLTHNGFFAKTTVPSQNGKEGEEEETAYALTPPSKLLVKGK
PTCLASIVRGALHPSSLDMWRSSEKWFNEDKELTLFESATGESFWDFLNKDSESGTLSMF
QEAMAADSQMFKLALKECRHVFEGLESLVDVGGGTGGVTKLIHEEFPHLKCTVFDQPQ
VVGNLSGNENLKFVGGDMFKSIPPADAVLLKWVLHDWNDELSLKILKNSKEAISGKGK
EGKVIIIDISIDEASGDRELTELQLDYDLVMLTMFNGKEREKKEWEKLISDAGFSSYKITPI
CGFKSLIEVFP*
>SEQ ID NO: 3 (GeCYP81E1) 
MEILSLLSYSVFYLALFFIFNIVIRARKFKNLPPGPPSLPIIGNLHHLKRPLHRTFKGLS
EKYGHVFSLWFGSRLVVVVSSASEFQQCFTKNDVVLANRPRFLSGKYIFYNYTTLGSTS
YGEHWRNLRRITALDVLSNHRINSFSGIRRDETQRLITRLADDSSTNFAEMELSSRLYDM
TFNNIMRMISGKRYYGEDCDTSDLQEASQFRDMVSELLQLSGANNKTDFMPLLRFLDFE
NLEKRLKDISGKTDAFLRGLIEEHRTKKERANTMIDHLLNLQDSQPEYYTDQIIKGLALA
MLLAGTDSSAVTLEWSMSNLLNHPEVLKKVKDELDTHVGQDRLVDESDLPKLTYLKNV
INETLRLYTPAPLLLPHSTSDECNIGGYKVPQDTIVLINAWAIHRDPELWTEATTFKPERFE
KKGELEKLIAFGMGRRACPGEGLAIRAISMTLALLIQCFDWKLINGDKIDLAERDGFTLT
KLVPLKAMCKSRPVINKVFKQ*
>SEQ ID NO: 4 (GgI2'H) 
MEILSLLSYSVFYVALFFICNLLFQARKFKNLPPGPPSLPIIGNLHHLKRPLHRTFKGL
SEKYGHVISLWFGSRLVVVVSSLSVFQECFTKNDIVLADRPRFLSGKYIFYNYTTVGSSSY
GEHWRNLRRITSLDVLSTHRINSFSGVRRDETQRLIQKLAEESSTDFAEIELTSKFYDMTF
 
NNIMRMISGKRYYGDDCDMMDMEEAKQFRAMVSELLQLSGANNKTDFMPVLRLVDFE
NLEKRLKKISSKTDTFLRGLIQEHRNKKQHTNTMVDHLLSLQESQPEYYTDQIIKGLALG
MLLAGTDSSAVTLEWALSCLLNHPEALKKARDELETHVGQDRLLEESDLTKLPYLKNIIY
ETLRLYTPAPLLLPHSSSDECIIGGFKVPRETIILINAWSIHRDPQIWSEATSFKPERFEKEGE
LDKLIAFGLGRRACPGEALALRGISLTLGLLIQCFEWKLVGDKEIDMREESGFTLSRLIPL
KAMCKARPVANKLVNTQAVSLGD*
>SEQ ID NO: 5 (MsIFR) 
MATENKILILGPTGAIGRHIVWASIKAGNPTYALVRKTPGNVNKPKLITAANPETKEE
LIDNYQSLGVILLEGDINDHETLVKAIKQVDIVICAAGRLLIEDQVKIIKAIKEAGNVKKFF
PSEFGLDVDRHDAVEPVRQVFEEKASIRRVIEAEGVPYTYLCCHAFTGYFLRNLAQLDAT
DPPRDKVVILGDGNVKGAYVTEADVGTFTIRAANDPNTLNKAVHIRLPKNYLTQNEVIA
LWEKKIGKTLEKTYVSEEQVLKDIQESSFPHNYLLALYHSQQIKGDAVYEIDPAKDIEASE
AYPDVTYTTADEYLNQFV*
>SEQ ID NO: 6 (GgIFR) 
MNMIEVGGYPGNPINTKQSNAQQHKPTASLTTYQNKKKKNTHPSFFNSPYFYFNFN
IPMAAENKILILGPTGAIGRHIVWASVKAGNPTFALVRKTNGPVNKPKLITAANPESKEQL
LESYQNAGVTLLEGDINDHESLVKAIKQVDVVICATGRLLIDDQVKIIAAIKEAGNVKRFF
PSEFGLDVDRHDSVEPVREVFEDKARIRRVIEAEGVPYTYLCCHAFTGYFLRNLAQLDAT
VPPRDKVVILGDGNVKGAYVTEADVGTYTIRAANDPRTLNKAVHIRLPANYLTANEVVS
LWEKKIGKTLEKTYVPEEKVLKDIQESSFPHNYLLALYHSQQIKGDAVYEIDPAKDVEAY
DLYSDVKYTTADEYLNQFV*
>SEQ ID NO: 7 (MsVR) 
MAEGKGRVCVTGGTGFLGSWIIKSLLENGYSVNTTIRADPERKRDVSFLTNLPGASE
KLHFFNADLSNPDSFAAAIEGCVGIFHTASPIDFAVSEPEEIVTKRTVDGALGILKACVNSK
TVKRFIYTSSGSAVSFNGKDKDVLDESDWSDVDLLRSVKPFGWNYAVSKTLAEKAVLEF
GEQNGIDVVTLILPFIVGRFVCPKLPDSIEKALVLVLGKKEQIGVTRFHMVHVDDVARAHI
YLLENSVPGGRYNCSPFIVPIEEMSQLLSAKYPEYQILTVDELKEIKGARLPDLNTKKLVD
AGFDFKYTIEDMFDDAIQCCKEKGYL*
>SEQ ID NO: 8 (GgVR) 
MAEGKGRVCVTGGTGFLGSWLIKRLLEDGYGVNTTIRSDPERKRDISFLTNLPGASE
RLKIFNADLSDPESFGPAIEGCVGVFHTASPIDFAVSEPEEIVTKRTVDGTLGILKACKNSK
 
TVKRVVYTSSGAAVSWGGTEKDVLDESDWSDVDMLRSVKPYSWSYAVSKTMTEKAVL
EFGEQHGLDVVTLILPFIVGRFICPKIPDSVEKALVLVLGKREQIGVTRFHMVHVEDVARA
YIFLLEHPNPKGRYNCSPFIVPIEEVSELLSAKYPEYQIPTVEELKEIKGAKLPDLKSKKLV
DAGFEFKYTIEDMFDDAIQCCKEKGYL*
>SEQ ID NO: 9 (PcM4DT) 
MDWGLAISSSSKAYSVTTGANLWRSKHVTNNIHYPSSCITKASQHKKKTQIEYNVL
RFQRPSLGHGYNFSGGGGSTYQECNRKYAVKAVYDQPKDFELEASNPKNILDSAKKFLA
AFYYFSYPYTMIGITLCAFCSSALAVEKLSDISLPFFIGVLQAVIPQLFIEIYLSGVNQLYDL
EIDKINKPHLPMASGQFSFKTGVIMSAAFLALSFGFTWLTGSWPLIWNLVVIASSWTAYSI
DVPFMRWKRYPLVAAMCMIATWGLALPISFFHHMQTFVLQRPIGFPRSLGFLVAFMTFYS
MGIALSKDIPDVEGDKEHGINSFSVLLGQKRIFWICVSLFEMAFGVGLLGGVTSPHFWTK
IITGLGNVILGSILWYQAKSFDLTDKASTGSFYMYIWKLLYAALLLMAFVR*
>SEQ ID NO: 10 (GgPT) 
MSSLFGSNVFESISTYRSYAPKASQIIKRKIQKEYNFLRSRQPSLKIIIYKGIEGGSTQQ
ECNRKYVVKTVPKPSFESDPRSIDQKDILECIKNFLDALYMFTTPYSFFSSALYIISVSFIVV
EKLSDISPLFFTGVLQAVVPNLFVHIYMTGLNQLCDVEIDKINKPYLPLVSGKISFATGVIV
VASCLFLSLWLGWIVGSWPSTCVVISIAMIWTAYSINVPLLRWKKYPVLAAMCIFLSLAV
VNPIGYFLHMQTFVFKRPASFSRPLIFVIAFKSFFSLAVALFKDVPDIEGDQTFGVQSFVAR
FGKKRVFWICISLIEMAYGFALLMGATSAFLWSKIVTVLGHAVLASFVLYRAKSIDLRSKT
SITSFYMLIWKLMYMEYFLMPLVR*
>SEQ ID NO: 11 (GgPTR) 
MAETTTSKSKILFIGGTGYIGKFIVEASVNAGHPTFVLIRDSTLSNPAKSPIIDKFKSLA
VNLVFGDLYDHQSLVKAIKQVDVVISTVGHLQLGDQDKIISAIKESGNVKRFFPSEFGND
VDRTHAVEPAKSAFATKAKIRRTIEAEGIPYTYVSSNFFAGYFLPTLSQPGATAAPRDKVII
LGDGNPKAVANKEEDIATYTIKAVDDPRTLNKILYIRPPANTLSFNELVSLWEKKIGKTLGR
VYVPEEQLLKQIQESSPPINVILSIGHSVYVKGDHTNFEIEPAFGVEASALYPDVKYTTVD
EYLNQFV*
>SEQ ID NO: 12 (GgPTR) 
MDSTAASKILVIGGTGYMGRFLVEASAKAGHPTFALVRHSTVTNPDKSSIIHSFNTLG
VNLLLGDINDHQSLVKAIKQADVVISTVNHQYISDQYKIISAIKEAGNIKRFFPSEFGNDV
VRTHGVDGAKALFDIKAKFRRTIEAEGIPHTYVVANFLTQHFLPTRSRLLAIAAPLDKVVI
 
LGDGNTKATFNTEEGVATFTIRAVDDPRTLNKILYIRPPANTLSYNDLVSLWEKKTGNTLE
RVYVPEEQVLRLIQESSYPLNMALSICHAAYVKGDHTNYEIEPSFGVEALELYPDVKFTT
VDDYLEQNRDCTPFYLNQLIPITNGTKF*
>SEQ ID NO: 13 (GgOC) 
MAFTYSTKKLALLSIITIFISSFPEPSTSVDLESGFLQCFSSGLGNSNSTAELVLTKNSSS
YASLLQSSIRNLRFMGTSVPKPTLIVTPRDLFHIQTSIKCSVKQGLQIRVRSGGHDYEGLSY
VSNDDVPFLIIDLTNLRSITIDIKDETAWVQSGATLGELYYAIAKKSNVHGFPAGSCPTVGV
GGHFSGGGFGTIFRKYGLAADNVIDAQMVDVNGKILKNRTLMGEDLFWAIRGGGGSSF
GVVTAWKVKLVIIVPPKVTVFNIPKTLDQNASTLFIIKWQIVADKLPGELFLIISVMGVSN
ANSISSSSGGGRTVLVSFTGLYLGTVENLLPLMQNNFAELGLQHNNCTEMSWIQSVLFFA
GYDPINDSLEVLLQRNQTFGSFKAKSDYVMEPIPTSGLEGLWNMLLEENSSPTLILTPYG
GRMSEISESETPFPHRNGSIYGIQYLVYWDSSEETPKHIAWMRRVYSYMVLYVSKFPRAA
YLNYRDLDIGVNRGNTSYDYEEAKSWGLKYFKCNFERLAKVKAEVDPSNFFRIIEQSIPL
LF*
>SEQ ID NO: 14 (NhDMT) 
MLVDTGLGLISELQAKLGWAVLLQIVPITIVAYNLLWFIYASFFSSLRKIPGPFLARISR
VWEMKKTATGNIHEIMMDLHRRHGAIVRIGPRRYDFDTMEALKIIYRIGNALPKADYYK
PFGLPSFPNLFDEQNPARHSAIKKQVASLYTMTALLSYEEGVDGQTAILKEQLQRFCDQK
QVIDLPRFLQYYAFDVIGVITVGKSMGMMESNSDTNGACSALDGMWHYASMMAYIPN
MHAWWLRLSSLLPIEVPIKGLTEYVERRIIQYRLKAAEFGDDALKGENNFLAKLLLME
KKGTVTPVETQQAVGLNIGAGSDTTANALSTILYYLYTNPRTLHTLREELERYVKDGPISF
QQSQSMPYLQAVIKEALRLHPGVGTQLTRVVPKGGLVIEGQFFPEGTEVGVNGWALYHN
KAIFGNDASIFRPERWLEANENINIGGSFAFGAGSRSCIGKNISILEMSKAIPQIVRNFDIEI
NHGDMTWKNECWWFVKPEYKAMIKPRRCCLSRDESLV*
>SEQ ID NO: 15 (GgDMT) 
MEHFYMSLLLLFVTLVSLSLFFLIFYHNKHNMNNNNNLPPGKMGYPVIGESLEFLS
MGWKGHPEKFIFDRMVRYSSELIKTSILGVPTVIFCGPACNKFLFSNENKLVTAWWPDSV
NKIFPTTSNSKEESKKMRKLLPQFLKPEALQRYVGIMDTLAQRHFASLWEEKTHVTVYP
LAKRYTFMLACRLFMSVEDENHVAKFREPFHLLASGIISVPIDLPWTPFNRGIKASNFIRK
ELLKIIRQRKVDLAQGVASPTQDILSHMLLTCDDENGEFMTELNIADKILGLLIGGHDTAS
AACTFIVKYLAELPHIYDRVYQEQMEIANSKSPGELLNWDDINKMRYSWNVASEVMRV
APPLQGGFREAINDFVFNGFSIPKGWKLYWSANSTHKNPEYFPAPEKFDPTRFEGNGPAA
 
YTFVPFGGGPRMCPGKEYARLEILVFMHNLVKRFKWEMLIPEEKIVVDPLPMPANDLPIR
LYPHNT*
>SEQ ID NO: 16 (tPcM4DT) 
ASQHKKKTQIEYNVLRFQRPSLGHGYNFSGGGGSTYQECNRKYAVKAVYDQPKDF
ELEASNPKNILDSAKKFLAAFYYFSYPYTMIGITLCAFCSSALAVEKLSDISLPFFIGVLQA
VIPQLFIEIYLSGVNQLYDLEIDKINKPHLPMASGQFSFKTGVIMSAAFLALSFGFTWLTGS
WPLIWNLVVIASSWTAYSIDVPFMRWKRYPLVAAMCMIATWGLALPISFFHHMQTFVLQ
RPIGFPRSLGFLVAFMTFYSMGIALSKDIPDVEGDKEHGINSFSVLLGQKRIFWICVSLFEM
AFGVGLLGGVTSPHFWTKIITGLGNVILGSILWYQAKSFDLTDKASTGSFYMYIWKLLYA
ALLLMAFVR*
>SEQ ID NO: 17 (GgOMT) 
ATGGACTTTTCGACAAATGGGTCTGAGGAAAGTGAACTTTATCATGCTCAAATTC
ACTTGTACAAGCATGTTTACAATTTTGTGTCTTCAATGGCTTTAAAGTCAGCCATGGA
GTTGGGCATAGCTGATGTCATACACAATCATGGTAAGCCAATCACCCTCCCCCAGCTG
GCATCTGCTCTGAAATTGCACCCTTCTAAGGTTGGAGTACTTTACCGATTCCTCCGGTT
GCTAACTCACAACGGCTTCTTTGCTAAAACCACTGTTCCTTCTCAGAATGGAAAAGA
AGGCGAGGAGGAGGAAACGGCTTATGCATTGACTCCACCATCAAAATTATTGGTAAA
AGGAAAGCCTACTTGTTTGGCGAGCATTGTTAGAGGTGCACTGCATCCTAGTAGCCTT
GACATGTGGAGAAGTTCCGAGAAGTGGTTCAATGAAGATAAAGAGCTTACGTTATTC
GAGTCAGCCACAGGAGAAAGTTTTTGGGATTTCTTGAACAAGGACAGCGAATCAGG
AACTCTCTCAATGTTTCAAGAAGCAATGGCAGCAGACTCTCAAATGTTCAAACTTGC
CTTGAAGGAATGCAGGCATGTGTTTGAGGGTTTGGAATCTCTTGTTGATGTGGGTGGA
GGAACTGGAGGGGTTACCAAGTTAATCCATGAAGAATTTCCCCATCTGAAATGCACA
GTATTTGATCAACCGCAAGTAGTGGGGAACCTAAGTGGAAATGAGAATCTGAAGTTT
GTTGGTGGTGATATGTTTAAGAGTATACCACCTGCTGATGCTGTCCTACTGAAATGGGT
TCTTCATGATTGGAATGATGAGCTTAGCCTCAAAATCCTTAAAAACTCCAAGGAGGCT
ATTTCTGGTAAAGGCAAAGAAGGGAAAGTGATAATCATTGACATATCGATTGATGAAG
CGTCAGGGGATAGGGAACTTACAGAATTGCAGTTGGATTATGATCTAGTGATGCTGAC
AATGTTCAATGGTAAGGAGCGTGAGAAAAAGGAATGGGAAAAGCTCATTAGTGACG
CAGGATTTTCCTCATATAAAATTACACCAATTTGTGGTTTCAAATCTTTAATTGAGGTC
TTTCCTTGA
>SEQ ID NO: 18 (GgI2'H) 
ATGGAAATATTGTCACTATTAAGTTATAGCGTGTTCTACGTGGCCCTGTTTTTCATC
TGCAACCTGTTGTTCCAAGCTCGCAAATTCAAAAACCTGCCACCGGGTCCGCCAAGC
CTGCCTATCATCGGTAATCTTCACCATCTGAAGCGTCCGCTGCACCGCACCTTCAAGG
GTTTGTCCGAAAAGTACGGCCACGTGATTAGCTTATGGTTTGGCAGCCGGCTGGTCGT
TGTTGTGAGCAGCCTGTCTGTTTTTCAGGAGTGCTTCACCAAGAACGATATTGTTTTG
GCGGATCGTCCGCGTTTCCTGTCTGGCAAATACATTTTTTACAATTATACGACCGTAGG
TTCTAGCAGCTATGGCGAACATTGGCGTAACTTGCGCCGTATCACCAGCCTGGATGTT
CTTTCCACCCACCGCATTAATAGCTTCAGCGGTGTTCGTCGTGACGAAACGCAGCGTT
TGATCCAGAAACTGGCGGAGGAGTCCTCGACCGACTTCGCCGAAATTGAATTGACTT
CGAAGTTTTATGATATGACCTTCAACAACATCATGCGCATGATCTCCGGCAAACGTTAT
TACGGCGACGACTGTGATATGATGGATATGGAAGAGGCGAAACAGTTTCGTGCTATGG
TGTCAGAACTCTTGCAACTGAGCGGTGCAAATAACAAGACCGACTTCATGCCGGTCC
TGCGTCTGGTGGATTTCGAAAACCTGGAAAAGCGCCTGAAGAAGATCAGCAGCAAA
ACCGACACCTTTCTGAGAGGCCTGATCCAAGAGCACCGCAACAAGAAGCAACACAC
CAATACCATGGTTGACCACCTGCTGTCCCTGCAAGAGTCTCAGCCGGAGTACTACACT
GACCAGATTATCAAAGGTTTAGCGTTAGGTATGCTGTTGGCGGGCACCGACAGCTCCG
CAGTCACCTTGGAGTGGGCACTGAGCTGCCTGCTGAATCATCCGGAGGCGCTTAAGA
AGGCGCGTGACGAGCTGGAAACGCACGTGGGCCAGGATCGTCTCTTAGAGGAGTCC
GACCTGACGAAACTGCCATATCTGAAGAACATTATTTACGAAACGCTGCGTTTATATAC
TCCGGCACCGCTGTTGCTGCCGCATTCTTCATCCGATGAATGTATTATTGGTGGTTTTA
AGGTGCCGCGTGAAACCATCATCCTGATCAACGCGTGGAGCATTCATCGTGATCCGCA
GATCTGGTCGGAAGCTACCAGTTTTAAACCGGAACGTTTCGAGAAGGAGGGTGAACT
GGACAAGCTGATCGCATTTGGTCTCGGTAGACGTGCCTGCCCGGGCGAGGCTTTGGC
CCTGCGCGGTATTAGCCTGACGCTGGGCCTGTTGATTCAATGTTTTGAGTGGAAACTG
GTTGGTGACAAAGAAATCGATATGCGCGAAGAGAGTGGCTTTACCCTGAGCCGTCTG
ATCCCGCTGAAAGCTATGTGCAAAGCTCGCCCGGTTGCGAACAAACTGGTAAATACC
CAGGCGGTGTCCCTCGGGGATTAA
>SEQ ID NO: 19 (GgIFR) 
ATGAACATGATTGAAGTCGGTGGTTACCCAGGTAACCCAATTAACACCAAACAAT
CTAATGCCCAACAACACAAGCCAACCGCTAGCTTGACTACTTACCAAAACAAGAAGA
AAAAGAACACTCATCCATCCTTCTTTAACTCTCCATACTTCTACTTCAACTTCAACATC
CCAATGGCTGCTGAAAACAAGATCCTGATCTTAGGTCCAACTGGTGCCATTGGACGTC
ATATCGTTTGGGCTTCCGTTAAAGCCGGTAACCCAACCTTTGCTTTGGTTAGAAAGAC
CAACGGTCCAGTTAACAAACCAAAGTTGATCACTGCTGCCAATCCAGAATCCAAGGA
 
ACAATTACTAGAATCTTACCAAAACGCTGGTGTCACTTTGTTGGAAGGTGACATTAAT
GACCACGAATCTTTGGTTAAGGCTATCAAGCAAGTCGATGTCGTCATCTGTGCTACCG
GTAGATTATTGATCGATGACCAAGTTAAGATCATTGCAGCCATCAAGGAAGCCGGTAA
CGTTAAGAGATTCTTCCCATCTGAATTCGGCTTGGACGTTGACAGACACGACTCTGTT
GAACCTGTCCGTGAAGTCTTCGAAGATAAGGCTAGAATTAGAAGAGTTATCGAAGCT
GAAGGTGTTCCATACACTTACTTATGTTGTCACGCTTTCACCGGTTACTTTTTGAGAAA
CTTGGCTCAATTGGATGCTACTGTTCCTCCAAGAGACAAGGTTGTTATTTTGGGTGAT
GGTAACGTCAAGGGTGCTTACGTTACTGAAGCTGACGTTGGTACATATACTATCAGAG
CTGCTAACGATCCAAGAACCTTGAACAAGGCTGTTCACATTAGATTGCCAGCTAACTA
TTTGACCGCCAACGAAGTTGTCTCCTTGTGGGAAAAGAAGATTGGTAAGACCTTGGA
AAAGACCTACGTCCCAGAGGAAAAAGTCTTGAAGGACATCCAAGAATCCTCTTTCCC
ACACAACTACTTGTTGGCCCTATACCACTCTCAACAAATTAAGGGTGACGCTGTCTAC
GAAATAGACCCAGCTAAGGATGTCGAAGCTTACGACTTGTACTCAGATGTAAAGTAC
ACCACTGCTGATGAATACTTGAACCAATTCGTCTGA
>SEQ ID NO: 20 (GgVR) 
ATGGCCGAGGGCAAGGGCCGGGTATGTGTCACGGGCGGCACCGGCTTCCTGGGA
TCGTGGCTGATCAAGCGTTTGCTCGAGGACGGCTACGGCGTCAACACCACGATCCGG
AGTGACCCGGAGCGCAAGCGGGACATCAGCTTCCTCACCAACCTGCCCGGGGCGAG
CGAGCGGCTGAAGATCTTCAACGCCGACCTGTCCGACCCCGAATCCTTCGGCCCCGC
GATTGAAGGCTGCGTGGGTGTCTTCCACACCGCCTCCCCGATCGACTTCGCCGTCTCG
GAGCCCGAGGAGATCGTGACCAAGCGCACCGTCGACGGGACGCTGGGCATCCTCAA
GGCCTGCAAGAACTCCAAGACCGTCAAGCGGGTCGTGTACACGTCCAGCGGCGCCG
CGGTCAGCTGGGGCGGGACCGAGAAGGACGTGCTCGACGAGAGCGACTGGTCGGAT
GTGGACATGCTGCGCTCGGTGAAGCCGTACTCCTGGTCCTACGCCGTGAGCAAGACG
ATGACCGAAAAGGCGGTGCTGGAGTTCGGCGAGCAGCACGGCCTGGACGTCGTCAC
CCTGATCCTGCCGTTCATCGTCGGCCGCTTCATCTGCCCCAAGATCCCCGACTCCGTC
GAGAAGGCGCTGGTCCTGGTGCTCGGCAAGCGCGAGCAGATCGGCGTGACCCGGTT
CCACATGGTCCACGTCGAGGACGTGGCCAGGGCCTACATCTTCCTGCTGGAGCACCC
GAACCCGAAGGGGCGCTACAACTGCTCCCCCTTCATCGTGCCGATCGAGGAGGTCTC
GGAGCTCCTCTCCGCGAAGTACCCAGAGTACCAGATCCCGACGGTGGAGGAACTCA
AGGAGATCAAGGGTGCCAAGCTCCCGGACCTCAAATCGAAGAAGCTGGTCGACGCG
GGGTTCGAGTTCAAGTACACCATCGAGGACATGTTCGACGACGCCATCCAGTGCTGC
AAGGAGAAGGGATACCTGTAA
>SEQ ID NO: 21 (GgPT) 
ATGTCTTCTTTATTCGGGTCAAATGTATTTGAGAGTATATCAACATATCGTAGCTAT
GCTCCAAAAGCCAGTCAACATAAAAGGAAGATTCAGAAAGAATATAATTTCCTTCGC
TCCCGACAGCCTTCATTAAAGCACATTTACAAGGGAATTGAGGGTGGAAGTACACAA
CAAGAGTGCAATAGGAAATATGTTGTAAAAACAGTGCCCAAGCCAAGCTTTGAATCA
GATCCTAGGTCTATAGATCAAAAGGACATCTTGGAATGCATTAAGAATTTTCTTGATGC
TTTATATATGTTTACTACTCCTTATTCTTTTTTCTCCTCAGCGTTGTACATTATCTCCGTT
TCTTTCATCGTTGTGGAGAAGCTTTCCGACATAAGCCCGCTGTTCTTCACTGGTGTGC
TCCAAGCAGTTGTTCCAAATCTTTTTGTTCACATATACATGACAGGACTAAACCAGCT
TTGTGATGTTGAAATTGACAAGATCAACAAACCCTACCTTCCCCTGGTCTCAGGAAA
AATTAGTTTTGCAACCGGGGTTATTGTTGTCGCGTCATGCCTGTTCCTATCGCTTTGGC
TTGGATGGATTGTGGGCTCTTGGCCATCAACTTGTGTCGTGATATCTATCGCTATGATT
TGGACTGCTTACTCTATCAATGTACCTCTCCTCAGATGGAAGAAGTATCCAGTCCTGG
CAGCAATGTGTATATTTTTATCTCTTGCTGTAGTGAACCCTATTGGTTATTTCTTGCATAT
GCAAACATTTGTGTTCAAGAGACCTGCTTCATTTTCAAGGCCGCTCATTTTCGTGATT
GCTTTCAAAAGTTTTTTTAGTTTGGCAGTGGCATTATTTAAGGATGTTCCTGATATTGA
AGGTGATCAGACCTTTGGCGTTCAGTCGTTTGTTGCCAGATTTGGAAAGAAAAGAGT
TTTCTGGATATGTATTTCTCTCATCGAAATGGCATATGGTTTTGCTTTGTTAATGGGGGC
TACTTCCGCTTTTCTATGGTCCAAAATAGTTACAGTATTGGGCCATGCTGTTCTTGCCA
GTTTTGTACTATATAGAGCAAAAAGCATTGATTTGCGTAGCAAAACGAGCATTACCTC
TTTTTACATGTTGATATGGAAACTGATGTATATGGAGTATTTTTTGATGCCATTGGTCCG
GTGA
>SEQ ID NO: 22 (GgPTR) 
ATGGCTGAAACAACTACCTCAAAATCAAAGATTCTTTTCATTGGAGGAACTGGCT
ACATTGGGAAATTTATTGTTGAAGCTTCTGTTAATGCTGGTCATCCAACTTTTGTGTTA
ATAAGAGATTCAACTCTGAGCAATCCAGCGAAGTCTCCCATAATTGATAAATTCAAGA
GTCTCGCAGTGAACCTCGTTTTTGGGGACTTGTATGATCACCAGAGTCTTGTGAAAGC
AATCAAACAAGTTGATGTTGTGATCTCCACCGTTGGACATCTTCAGCTTGGAGATCAA
GATAAGATAATATCAGCCATTAAAGAGTCTGGTAATGTTAAGAGGTTTTTCCCTTCTGA
ATTTGGGAATGATGTCGACCGTACACATGCTGTAGAGCCCGCTAAGTCTGCCTTTGCA
ACCAAGGCAAAAATCCGCAGGACAATAGAGGCCGAGGGAATTCCATACACTTACGTA
TCGTCCAACTTCTTTGCTGGATATTTCTTGCCTACTCTATCTCAACCTGGTGCAACTGC
CGCTCCAAGAGACAAGGTTATAATCTTAGGGGATGGAAACCCTAAAGCTGTCGCTAA
CAAGGAGGAAGATATTGCTACATATACAATCAAGGCGGTGGATGATCCAAGAACATTG
 
AACAAAATACTTTATATTCGGCCGCCGGCAAATACTTTATCTTTCAATGAATTGGTGAG
TCTGTGGGAAAAGAAAATTGGTAAAACTCTTGGCCGAGTTTATGTGCCAGAAGAGCA
GCTCTTGAAGCAGATTCAAGAATCGAGTCCTCCAATTAATGTCATACTAAGCATCGGC
CACTCAGTATATGTTAAAGGTGATCATACAAATTTTGAGATTGAGCCTGCATTTGGTGT
CGAAGCATCTGCACTGTATCCTGACGTAAAGTACACGACGGTTGACGAATATTTGAAT
CAATTTGTTTGA
>SEQ ID NO: 23 (GgOC) 
ATGGCTTTTACGTACAGTACCAAGAAACTTGCACTGCTCTCAATCATTACTATTTT
CATCTCCAGCTTTCCTGAACCATCTACATCGGTTGACCTTGAGTCAGGCTTCCTTCAAT
GTTTTAGTTCAGGATTGGGCAACAGTAACAGCACAGCGGAGTTGGTGCTAACAAAAA
ATTCTTCCTCTTATGCTAGCCTGCTTCAGTCTAGTATTAGAAACTTGCGGTTCATGGGA
ACATCTGTACCGAAGCCTACATTGATAGTGACTCCTAGAGATCTCTTTCATATCCAAAC
AAGCATCAAATGCTCAGTTAAACAAGGATTACAGATCCGTGTAAGGTCCGGGGGACA
TGATTACGAAGGACTCTCCTACGTTTCTAATGATGATGTGCCATTTTTGATAATTGATCT
TACTAATCTGCGAAGTATTACCATTGACATTAAGGATGAAACCGCTTGGGTCCAAAGT
GGAGCCACTCTTGGAGAGCTATATTATGCAATTGCTAAGAAGTCGAATGTTCACGGCT
TCCCTGCTGGTTCATGCCCTACGGTAGGGGTTGGGGGCCATTTTTCAGGTGGTGGATT
TGGAACTATATTTAGAAAGTACGGGCTGGCCGCTGATAATGTGATTGATGCTCAAATG
GTGGATGTTAATGGAAAGATACTTAAGAATAGGACATTGATGGGTGAAGATTTGTTTT
GGGCAATACGGGGGGGTGGTGGCTCATCATTTGGGGTCGTTACTGCTTGGAAAGTTA
AGTTAGTTCATGTTCCTCCAAAAGTAACTGTCTTCAATATCCCCAAAACACTAGATCA
AAATGCAAGCACCCTTTTCCACAAATGGCAAATTGTTGCAGATAAATTGCCAGGAGA
ACTATTCTTGCATTCCGTGATGGGCGTCTCTAATGCCAATTCTATTTCTTCTTCCTCGGG
TGGAGGAAGAACAGTGCTTGTCAGCTTTACTGGATTATATCTGGGAACTGTTGAAAAT
CTCTTACCTCTAATGCAGAACAATTTTGCTGAATTGGGTCTTCAACACAATAACTGTAC
AGAAATGAGTTGGATTCAGAGCGTACTTTTCTTTGCAGGATATGATCCAATCAACGAT
TCTTTAGAAGTACTCCTGCAGAGGAACCAGACATTTGGTTCTTTCAAAGCAAAAAGC
GACTATGTGATGGAGCCCATACCAACTTCAGGGTTGGAGGGCCTTTGGAATATGTTAT
TGGAAGAGAATAGTAGTCCAACTTTGATTCTCACGCCATATGGTGGTAGGATGTCTGA
GATAAGTGAGTCTGAAACTCCATTTCCTCATCGTAATGGTTCAATTTATGGTATACAGT
ATCTTGTTTATTGGGATTCATCTGAGGAAACTCCCAAGCATATTGCATGGATGAGGAG
AGTTTATTCTTACATGGTACTTTATGTATCAAAGTTTCCGAGGGCGGCTTATTTGAATTA
CAGAGACTTAGACATCGGTGTTAACCGAGGGAATACCTCCTACGACTATGAAGAAGC
CAAATCGTGGGGTCTGAAATACTTCAAATGTAACTTTGAGAGATTGGCTAAGGTGAA
 
GGCAGAGGTTGATCCTTCAAACTTCTTCCGCCATGAACAATCCATTCCTCTCCTGTTTT
GA
>SEQ ID NO: 24 (GgDMT) 
ATGGAGCATTTCTACATGTCTCTACTACTTTTGTTTGTAACACTGGTAAGCCTTTC
TCTCTTTTTCTTGATTTTCTATCATAACAAGCACAATATGAATAATAACAACAATCTACC
CCCTGGGAAGATGGGTTATCCTGTTATTGGAGAATCTCTTGAGTTTTTGTCAATGGGAT
GGAAGGGGCATCCTGAGAAGTTCATATTTGACAGGATGGTTCGTTACAGTAGTGAGTT
GATCAAAACATCAATCTTAGGAGTCCCAACTGTGATCTTTTGTGGACCAGCTTGTAAT
AAGTTCCTTTTCAGTAATGAGAACAAATTGGTCACTGCTTGGTGGCCAGATTCTGTAA
ATAAGATATTTCCCACGACTTCAAATAGCAAAGAGGAATCCAAGAAAATGCGGAAAT
TGCTTCCTCAATTTCTTAAGCCTGAAGCTTTACAGAGGTATGTTGGCATAATGGATACT
CTGGCTCAGCGACATTTTGCTAGTTTGTGGGAGGAGAAAACTCATGTTACTGTTTACC
CGCTGGCAAAACGGTACACATTCATGCTTGCTTGCCGCCTATTCATGTCTGTTGAAGA
TGAGAATCATGTGGCGAAGTTTCGCGAACCTTTCCACTTGCTTGCATCTGGTATCATAT
CAGTCCCAATTGATCTACCATGGACACCATTTAACAGAGGCATCAAGGCATCAAATTT
TATTAGGAAGGAACTGTTAAAGATAATTAGACAGAGAAAAGTAGATTTGGCCCAGGG
CGTAGCGTCCCCTACTCAAGATATTTTAAGCCATATGTTGTTAACATGTGATGATGAAA
ACGGTGAATTTATGACAGAATTGAATATTGCTGATAAAATACTCGGTCTCCTCATTGGT
GGTCATGACACAGCATCTGCCGCATGCACTTTCATCGTGAAGTATTTGGCTGAACTTC
CACACATTTATGATAGAGTTTATCAAGAACAAATGGAAATAGCTAATTCAAAATCTCC
AGGAGAGCTGCTTAATTGGGATGACATTAACAAGATGCGTTATTCATGGAACGTTGCT
AGCGAAGTCATGAGGGTGGCCCCTCCTCTTCAAGGGGGTTTTCGAGAAGCAATTAAT
GATTTTGTGTTCAATGGATTTTCGATTCCCAAGGGTTGGAAACTCTATTGGAGTGCAA
ACTCCACGCATAAAAATCCCGAGTATTTTCCAGCCCCGGAGAAATTCGATCCAACCAG
GTTTGAAGGAAATGGCCCTGCTGCATACACCTTTGTTCCATTTGGAGGAGGGCCAAG
AATGTGTCCTGGAAAAGAATATGCTAGATTAGAGATATTGGTCTTTATGCATAATCTTG
TTAAGAGATTCAAATGGGAAATGCTAATTCCAGAAGAAAAAATCGTTGTGGACCCGT
TACCTATGCCAGCAAATGACCTGCCCATTCGTCTTTACCCTCACAACACCTGA
>SEQ ID NO: 25 (PlOMT9) 
ATGGGTTCCAACAACGGTCGTAAAGCTTCTGAAATTTTCCAAGGTCAAGCTTTGC
TATACAGACACATGTTTGCTTTCGTCGACTCCATGTGTTTGAAGTCCATTGTCGAATTA
GGTATCCCAAACATCATTCACAAGCATGGTCAACCAATCACCTTATCCGAATTGATGTC
CATCTTGCAAGTCCCTCCAGCTAAGGTTGGTCACGTTCAATCTTTGATGAGATATCTG
 
GCTCACCACGGTTTCTTCGAAAGATTGAGAATTCACGAAAAGGATGCTTACGCTCTG
ACTGCTGCTTCCGAATTGTTAGTCAAGGGTACCGAACCATACTTGGCTCCAATGGTTG
AATGTATGTTGGATCCAACTTTGTCTGCTTCTTTCCACCAAATGAAGAAGTGGGTTTA
CGAAGAAGATTTGTCAGTTTTCGACATCTCCTTGGGTTCTAATTTATGGGATTTCTTGA
CCAAGAACCCAGCCTACAACGAAATCTTCAACGAAGCTATGGCTTCTGATTCTCAAAT
GTCTAACTTGGCCTTGAGAGATTGCAAGTTAGTCTTTGAAGGTTTGGAATCCATCGTG
GATGTCGGTGGTGGTACTGGTACTACAGCCAAAATTATTTGTGAAGCCTTCCCAAACT
TGAAGTGTATCGTCTTTGACAGACCACAAGTTGTTGAAAACTTGTTAGAGAACAACA
ACTTGACCTACGTTGGTGGTGACATGTTCAAGTCTATTCCAAAGGCCAACGCTGTCTT
ATTGAAATGGATTCTACATGACTGGACTGACAAGGACTGTCGTAAGATTTTGGAAAAC
TGTAAGGAAGCTATCTCTAACAATAGTAAGAGAGGTAAGATCATCATCATTGACATTG
TTATCAACGAAAAGCAAGACGAGCACAAGGTCACTGAATTGAAATTGTTGTTCGATG
TTGCTATGGCCTGTGTTTTGAACGGTAAGGAAAGAAACGAAGAAGAATGGAAGAAG
CTTTTCATGGAAGTTGGATTGCAAGACTACAAAATTTCTCCATTGACCGGTTACTTGT
CTTTGATAGAAATCTACCCATGA
>SEQ ID NO: 26 (GmCYP81E1) 
ATGGAAATTTTGTCTTTGTTGTCCTACTCAGTTTTCTATTTGGCTTTGTTCTTCATC
TTCAACATCGTTATCAGAGCTAGAAAGTTCAAGAATTTGCCTCCAGGTCCACCATCTT
TGCCAATTATCGGTAACCTCCACCACTTAAAGAGACCATTGCATAGAACCTTCAAGGG
TTTGAGTGAAAAGTACGGTCACGTTTTCTCCTTGTGGTTCGGTTCCAGACTGGTCGTC
GTTGTCTCCTCCGCCTCTGAATTCCAACAATGTTTCACCAAGAACGATGTTGTTCTAG
CCAACAGACCAAGATTCCTAAGCGGTAAATACATTTTCTACAACTACACCACTTTGGG
TTCTACCTCTTACGGCGAACATTGGAGAAACTTACGTAGAATCACTGCTTTGGATGTC
TTGTCCAACCATAGAATTAACTCTTTCTCTGGTATCAGAAGAGACGAAACTCAAAGAT
TGATCACCAGATTAGCTGATGACTCTTCCACCAACTTCGCTGAAATGGAATTGTCTTCT
AGATTGTACGACATGACTTTCAACAATATCATGAGAATGATCTCCGGTAAGAGATACTA
TGGCGAAGACTGTGATACTTCTGACTTGCAAGAAGCTTCTCAATTCAGAGACATGGTT
TCCGAATTGTTGCAATTGTCTGGTGCTAACAACAAAACTGATTTCATGCCATTGTTGA
GATTCTTGGACTTCGAAAACTTGGAAAAAAGATTGAAGGACATTTCTGGTAAGACCG
ATGCTTTTTTGAGAGGATTGATTGAAGAACACAGAACTAAGAAGGAAAGAGCCAAC
ACCATGATTGACCACTTGTTAAATTTACAAGATTCCCAACCAGAATACTACACCGACC
AAATCATCAAGGGTTTAGCCTTGGCCATGTTGTTGGCTGGTACTGACTCCTCTGCTGT
CACTTTGGAATGGTCCATGTCTAACTTGTTGAACCACCCAGAAGTTTTGAAGAAAGTT
AAGGATGAATTGGACACTCACGTCGGTCAAGATCGTTTAGTCGACGAATCTGATTTGC
 
CAAAGTTGACTTACTTGAAGAACGTTATCAACGAAACCTTAAGATTATACACTCCAGC
TCCATTGTTGCTGCCACACTCTACTTCCGATGAATGTAACATTGGTGGTTACAAGGTTC
CTCAAGACACCATTGTTTTGATCAACGCTTGGGCTATTCACCGTGATCCAGAATTATG
GACTGAAGCTACCACATTTAAGCCAGAACGTTTTGAAAAGAAAGGTGAATTGGAAA
AGTTGATTGCTTTCGGTATGGGTAGAAGAGCTTGTCCAGGTGAAGGTCTAGCCATTCG
TGCAATCTCTATGACCTTGGCTTTGTTGATTCAATGCTTTGACTGGAAGTTAATCAACG
GTGACAAGATCGACTTGGCTGAGAGAGATGGTTTCACTTTGACCAAATTGGTCCCATT
GAAGGCTATGTGTAAGTCTCGTCCAGTTATTAACAAGGTCTTTAAGCAATGA
>SEQ ID NO: 27 (MsIFR) 
ATGGCTACCGAAAACAAGATCTTGATTTTGGGTCCAACTGGTGCCATCGGTAGAC
ACATTGTCTGGGCTTCCATCAAGGCTGGTAACCCTACCTACGCCTTAGTCAGAAAGAC
CCCAGGTAATGTTAACAAGCCAAAGTTGATCACTGCTGCTAACCCAGAAACCAAAGA
AGAATTGATTGACAACTACCAATCTTTGGGTGTCATCTTGTTGGAAGGTGACATCAAC
GACCACGAAACATTGGTCAAGGCTATTAAGCAAGTCGATATTGTTATCTGTGCTGCCG
GCCGTTTATTGATCGAAGACCAAGTCAAGATAATCAAAGCTATCAAGGAAGCAGGTA
ACGTCAAGAAGTTCTTCCCATCTGAATTCGGTTTAGACGTTGACAGACATGATGCTGT
TGAACCAGTTAGACAAGTTTTTGAAGAAAAAGCTTCCATTAGAAGAGTTATTGAAGC
TGAAGGTGTTCCATACACTTATTTGTGTTGTCACGCTTTCACCGGTTACTTCTTGAGAA
ACTTGGCTCAATTGGACGCCACCGACCCTCCAAGAGACAAGGTTGTTATTTTAGGTGA
TGGTAACGTTAAGGGTGCTTACGTTACTGAAGCTGATGTTGGTACTTTTACTATCCGTG
CTGCTAATGACCCAAACACTTTAAACAAAGCCGTCCATATCAGATTGCCAAAGAACTA
CTTGACTCAAAACGAAGTCATTGCTTTGTGGGAAAAGAAGATTGGTAAGACCTTGGA
ΛΛΛGΛCTTΛCGTCTCΛGΛΛGΛΛCΛΛGTTTGΛΛGGΛTΛTCCΛΛGΛΛTCTTCTTTCCCΛ
CACAACTATCTACTGGCTTTGTACCACTCTCAACAAATCAAGGGTGATGCTGTATACG
AAATTGATCCAGCTAAGGACATTGAAGCCTCCGAGGCTTACCCAGACGTCACCTACA
CCACTGCCGATGAATACTTGAACCAATTCGTCTGA
>SEQ ID NO: 28 (MsVR) 
ATGGCCGAAGGTAAAGGTAGAGTCTGTGTCACCGGTGGTACTGGCTTCTTGGGT
TCCTGGATTATAAAGTCTTTATTAGAAAACGGTTACTCCGTCAACACCACTATCAGAG
CAGATCCAGAGAGAAAGAGAGATGTCAGTTTCTTGACTAATTTGCCAGGTGCCTCTG
AAAAATTGCACTTCTTCAACGCCGACTTGTCTAACCCAGATTCTTTTGCTGCTGCTATT
GAAGGTTGTGTTGGTATCTTCCACACTGCTAGCCCAATCGACTTTGCTGTTTCCGAAC
CAGAAGAAATCGTTACCAAGAGAACCGTTGACGGTGCTCTTGGAATCTTGAAAGCTT
 
GTGTTAACTCTAAGACTGTCAAGCGTTTCATTTACACCTCTTCTGGTTCTGCTGTTTCT
TTCAATGGTAAGGACAAGGATGTTTTGGACGAATCCGACTGGTCTGACGTCGACTTG
TTGAGATCTGTCAAGCCATTCGGTTGGAACTACGCTGTCTCCAAGACCCTAGCTGAA
AAGGCTGTATTGGAATTCGGTGAACAAAACGGTATTGACGTTGTTACTTTGATTTTGC
CTTTCATTGTCGGTCGTTTCGTCTGCCCTAAGTTGCCAGACTCCATTGAAAAGGCTTT
GGTCCTAGTTTTGGGTAAGAAAGAACAAATTGGTGTTACCAGATTTCACATGGTTCAC
GTTGATGACGTCGCCAGAGCCCATATCTATTTATTGGAAAACTCCGTTCCAGGTGGTA
GATACAACTGTTCTCCATTCATCGTTCCAATTGAAGAAATGTCACAATTGTTGTCCGCT
AAGTACCCAGAATACCAAATCTTGACTGTGGATGAATTGAAGGAAATCAAGGGTGCT
AGATTACCAGATTTGAACACTAAGAAGTTAGTCGATGCTGGTTTCGACTTCAAGTACA
CAATCGAAGACATGTTCGATGATGCTATCCAATGTTGTAAGGAAAAGGGTTACTTGTG
A
>SEQ ID NO: 29 (tPcM4DT) 
GCATCTCAACACAAGAAGACTCAAATCGAATACAACGTCTTGAGATTCCAA
AGACCATCCTTGGGTCACGGATACAACTTCTCCGGTGGTGGCTCCACTTACCAA
GAATGTAACAGAAAGTACGCCGTTAAGGCTGTCTACGACCAACCAAAGGACTTCGAA
TTGGAAGCTTCCAACCCAAAGAACATTTTGGACTCTGCCAAGAAATTCTTGGCTGCT
TTTTACTACTTCAGTTACCCATACACCATGATTGGTATCACTTTGTGCGCTTTCTGTTCC
TCTGCTTTAGCTGTCGAAAAGTTGTCTGATATCTCCCTCCCTTTCTTCATCGGTGTCTT
GCAAGCTGTTATCCCACAATTGTTTATTGAAATTTACTTGTCTGGTGTTAACCAATTATA
TGACTTGGAAATCGACAAGATTAACAAGCCACATTTGCCAATGGCTTCTGGTCAATTC
TCTTTCAAGACCGGTGTCATCATGTCTGCCGCCTTTTTGGCTTTGTCCTTCGGTTTCAC
CTGGTTGACCGGATCTTGGCCATTGATCTGGAACTTGGTTGTTATTGCCTCCTCCTGGA
CTGCTTACTCTATTGACGTTCCATTCATGAGATGGAAGAGATACCCATTAGTTGCTGCT
ATGTGTATGATCGCTACCTGGGGTCTGGCTTTACCAATCTCTTTTTCCACCACATGCA
AACTTTCGTTTTGCAACGTCCTATTGGTTTCCCAAGATCTTTGGGTTTCTTGGTTGCTT
TCATGACTTTTTATTCCATGGGTATTGCTTTGAGCAAGGATATTCCAGATGTTGAAGGT
GACAAGGAACATGGTATCAATTCTTTCTCAGTCTTGTTGGGTCAAAAGCGTATCTTCT
GGATCTGTGTCTCATTGTTTGAAATGGCTTTCGGTGTTGGTCTATTAGGTGGTGTTACT
TCTCCACACTTCTGGACCAAGATCATTACCGGTCTAGGTAACGTCATTTTGGGTTCCAT
CTTGTGGTACCAAGCTAAGTCTTTCGATTTGACAGATAAAGCTTCTACTGGTTCTTTCT
ACATGTACATCTGGAAATTGTTGTACGCTGCCTTATTGTTGATGGCCTTCGTCAGATGA
>SEQ ID NO: 30 (GgPTR) 
ATGGCCGAAACCACCACTTCTAAGTCTAAAATATTGTTTATTGGTGGTACCGGTTA
CATTGGCAAGTTCATTGTTGAAGCCAGTGTCAATGCCGGTCACCCAACTTTCGTCTTG
ATTAGAGATAGCACTTTGTCTAACCCAGCGAAGTCCCCAATCATTGATAAATTCAAGT
CTTTGGCTGTCAACTTGGTTTTCGGTGACTTGTACGACCACCAATCTTTGGTCAAGGC
CATCAAACAAGTTGATGTAGTGATCTCCACAGTCGGTCATTTGCAATTGGGTGACCAA
GACAAGATTATTTCCGCTATCAAGGAATCCGGTAATGTCAAGAGATTCTTCCCATCAG
AATTCGGTAACGACGTTGACAGAACCCACGCTGTCGAACCAGCTAAATCCGCTTTCG
CTACCAAGGCTAAGATCAGACGTACTATCGAAGCTGAAGGTATCCCATACACTTACGT
TTCTTCCAACTTTTTTGCTGGTTACTTCTTGCCAACCTTATCCCAACCAGGTGCTACTG
CTGCTCCAAGAGACAAGGTTATCATCTTGGGTGACGGTAACCCAAAGGCTGTTGCTA
ACAAGGAAGAAGATATTGCTACTTACACTATCAAGGCAGTCGATGACCCACGTACCTT
AAACAAGATTTTGTACATCAGACCACCAGCCAACACTCTATCTTTCAACGAATTGGTT
TCCTTATGGGAAAAGAAGATCGGTAAGACCTTGGGTAGAGTCTATGTCCCAGAAGAA
CAACTGTTGAAGCAAATTCAAGAGTCTTCTCCACCTATCAACGTTATCTTATCTATTGG
TCACTCTGTTTACGTCAAGGGTGACCACACCAACTTCGAAATTGAACCTGCTTTCGGT
GTTGAAGCCTCTGCTTTGTATCCAGATGTTAAGTACACCACTGTTGATGAATACTTGA
ACCAATTCGTTTGA
>SEQ ID NO: 31 (GgOC) 
ATGGCTTTCACTTACTCCACTAAGAAGTTGGCTTTGTTGTCTATCATCACTATATTC
ATCTCCTCCTTCCCAGAACCATCTACTTCTGTTGATTTGGAATCTGGTTTCTTGCAATG
TTTCTCTTCTGGTCTAGGTAACTCTAACTCAACTGCTGAATTGGTCTTGACCAAGAAC
TCTTCCTCTTACGCCTCCCTGTTGCAATCTTCCATTCGTAATCTGAGATTCATGGGTAC
CTCGGTCCCAAAGCCAACCTTGATTGTCACTCCACGGGATTTGTTCCATATTCAAACC
TCTATTAAGTGTTCTGTTAAGCAAGGTTTGCAAATCAGAGTTAGATCAGGTGGTCACG
ACTACGAAGGTTTGTCCTACGTCTCCAACGATGACGTTCCATTCCTGATTATCGACTTG
ACCAACTTGCGTAGTATCACCATTGACATCAAGGACGAAACTGCTTGGGTTCAATCTG
GTGCAACTTTGGGTGAATTGTACTACGCTATTGCCAAGAAGTCTAACGTCCACGGTTT
CCCAGCTGGTTCTTGTCCAACCGTCGGTGTTGGTGGTCACTTCTCTGGTGGTGGTTTC
GGTACCATCTTCAGAAAGTACGGTCTAGCCGCTGATAACGTTATCGACGCTCAAATGG
TTGATGTCAACGGTAAGATCTTGAAGAACAGAACCTTGATGGGTGAAGACTTGTTCT
GGGCTATCAGAGGCGGTGGTGGTTCTTCCTTTGGTGTCGTCACCGCTTGGAAGGTTA
AATTGGTCCATGTTCCACCAAAAGTGACAGTTTTCAACATCCCTAAGACCTTGGACCA
AAACGCTTCTACCTTGTTCCACAAGTGGCAAATCGTTGCTGACAAGTTACCAGGTGA
ATTGTTTTTGCACAGTGTCATGGGTGTTTCCAACGCCAACTCCATTTCCTCATCTTCCG
 
GTGGCGGTCGTACTGTCTTAGTTTCTTTCACCGGCTTGTACTTGGGTACTGTTGAGAA
CCTATTGCCATTGATGCAAAACAACTTTGCTGAATTAGGTTTACAACACAACAACTGT
ACTGAAATGTCCTGGATTCAATCCGTTTTATTCTTTGCCGGTTACGACCCAATCAACGA
CTCTTTGGAAGTTTTGTTACAAAGAAACCAAACTTTCGGTTCCTTCAAGGCCAAGTCT
GACTACGTTATGGAACCTATTCCAACTAGCGGGTTGGAAGGTTTGTGGAACATGTTGC
TCGAAGAAAATAGCTCTCCAACTTTGATCTTGACTCCATACGGTGGTAGAATGTCTGA
AATTTCTGAATCTGAAACCCCGTTCCCACACAGAAATGGTTCCATCTACGGTATCCAA
TACTTAGTTTACTGGGATTCCTCCGAAGAAACCCCAAAGCATATTGCTTGGATGAGAA
GAGTCTACTCTTACATGGTTTTGTACGTTTCTAAGTTCCCAAGAGCTGCTTACTTGAAC
TACAGAGATTTGGATATTGGTGTTAACAGAGGTAACACTTCCTATGACTATGAAGAAG
CCAAATCTTGGGGTTTAAAGTATTTCAAATGCAATTTCGAAAGATTGGCTAAAGTCAA
GGCTGAAGTCGATCCTTCTAACTTCTTCAGACACGAACAATCTATTCCATTGTTATTTT
GA
>SEQ ID NO: 32 (NhDMT) 
ATGCTCGTTGACACTGGTTTAGGATTGATTTCTGAATTGCAAGCCAAGTTGGGTT
GGGCTGTTTTGTTGCAAATTGTCCCGATCACCATCGTCGCTTACAACTTACTGTGGTTC
ATCTACGCTTCCTTCTTTTCCTCTTTGAGAAAGATTCCAGGTCCATTCTTGGCTCGTAT
CTCCCGTGTCTGGGAAATGAAGAAGACTGCTACCGGTAACATTCACGAAATCATGAT
GGACTTGCACAGACGTCACGGTGCTATCGTCAGAATCGGTCCAAGGAGATATGACTT
CGACACCATGGAAGCTTTGAAGATCATTTACAGAATTGGTAATGCCTTGCCTAAAGCT
GACTACTACAAGCCATTCGGTTTGCCATCTTTCCCAAACTTGTTTGATGAACAAAACC
CAGCTAGACATTCCGCTATCAAGAAACAAGTTGCTTCTTTGTACACTATGACTGCTTTA
TTGTCTTACGAAGAAGGTGTCGATGGTCAAACCGCCATCTTGAAGGAACAATTGCAA
AGATTCTGTGACCAAAAGCAAGTCATTGATTTGCCAAGATTCTTGCAATACTACGCTT
TCGATGTTATCGGTGTTATCACTGTTGGTAAGTCTATGGGTATGATGGAATCCAACTCT
GATACTAATGGTGCTTGCTCTGCTTTGGACGGTATGTGGCACTACGCCTCCATGATGGC
TTACATCCCTAACATGCACGCTTGGTGGTTGAGATTGAGCTCTCTTTTGCCAATCGAA
GTTCCAATTAAGGGTTTGACTGAATACGTTGAAAGAAGAATCATCCAATACAGATTAA
AGGCTGCTGAATTCGGTGATGATGCTGCCTTGAAAGGTGAAAACAACTTTTTGGCTA
AGTTGTTGTTGATGGAAAAGAAGGGTACAGTCACTCCAGTGGAAACTCAACAAGCT
GTCGGCTTGAACATTGGTGCTGGTTCTGACACTACTGCGAACGCTTTGAGTACCATCT
TGTATTATTTATACACCAACCCAAGAACCCTGCACACTTTGAGAGAAGAGTTGGAACG
TTACGTCAAGGATGGTCCAATCTCTTTCCAGCAATCTCAATCCATGCCATACTTGCAAG
CTGTCATCAAGGAAGCCCTAAGATTACATCCAGGTGTTGGTACCCAATTGACCAGAGT
 
TGTTCCAAAGGGTGGTTTAGTCATTGAAGGTCAATTCTTCCCAGAAGGTACCGAAGT
CGGTGTTAACGGTTGGGCTCTATACCACAACAAGGCCATTTTTGGTAACGATGCCTCC
ATCTTCAGACCAGAAAGATGGTTAGAAGCTAACGAAAACATTAACATCGGTGGTTCT
TTCGCCTTCGGTGCCGGTTCCAGATCTTGTATCGGCAAGAATATCAGCATTTTGGAAAT
GTCCAAGGCTATTCCACAAATTGTTAGAAACTTCGACATTGAAATTAACCACGGTGAC
ATGACCTGGAAGAACGAATGTTGGTGGTTCGTTAAACCAGAATACAAGGCTATGATTA
AGCCAAGAAGATGTTGTTTGTCTAGAGACGAATCTCTGGTTTGA
>SEQ ID NO: 33 (GgOMT) 
ATGGACTTCTCTACCAACGGTTCTGAAGAATCAGAATTATACCACGCTCAAATCC
ATTTATATAAGCACGTTTACAACTTCGTTTCTTCCATGGCTCTGAAGTCTGCTATGGAA
TTGGGTATTGCCGATGTTATTCACAATCACGGTAAGCCAATTACTTTGCCACAATTGGC
TAGTGCTTTGAAGTTGCACCCATCTAAGGTCGGCGTTTTATACAGATTCTTGAGATTAT
TGACTCACAATGGTTTTTTCGCTAAGACCACTGTTCCGTCACAAAACGGTAAGGAAG
GTGAGGAAGAAGAAACCGCTTACGCTTTGACCCCACCTTCCAAATTGTTGGTCAAGG
GTAAGCCAACTTGTTTGGCCTCCATTGTCAGAGGTGCTTTGCATCCAAGCTCTCTTGA
CATGTGGAGATCTTCTGAAAAGTGGTTTAACGAAGACAAGGAATTAACATTGTTCGA
ATCCGCCACTGGTGAATCTTTCTGGGATTTCTTGAACAAGGACTCCGAATCTGGTACT
TTGTCCATGTTCCAAGAAGCCATGGCTGCTGACTCTCAAATGTTCAAGTTGGCCTTGA
AGGAATGTAGACACGTCTTTGAAGGTTTGGAATCTTTAGTCGATGTTGGTGGAGGTAC
CGGTGGTGTCACCAAGTTGATCCACGAAGAATTCCCACATTTGAAATGCACCGTCTTC
GACCAACCACAAGTTGTTGGTAACTTGTCTGGTAACGAAAACCTAAAATTCGTCGGT
GGTGACATGTTTAAGTCCATCCCACCAGCTGATGCTGTCTTGTTGAAATGGGTTTTGC
ACGACTGGAACGATGAACTATCTTTGAAGATTTTGAAGAACTCCAAGGAAGCTATCT
CCGGTAAGGGTAAAGAAGGTAAGGTTATCATCATTGATATTTCTATCGACGAAGCTTC
CGGTGATCGTGAATTGACTGAATTGCAATTGGACTACGATTTGGTTATGTTGACCATGT
TCAACGGTAAGGAAAGAGAAAAGAAGGAATGGGAAAAATTAATCTCTGACGCTGGT
TTCTCCTCTTACAAGATCACTCCAATCTGTGGTTTCAAGTCCTTGATTGAAGTTTTCCC
ATGA
>SEQ ID NO: 34 (GgI2'H) 
ATGGAAATCTTGTCCTTGTTGTCTTACTCCGTTTTTTACGTTGCTTTGTTCTTCATC
TGTAACTTGTTATTCCAAGCTAGAAAGTTCAAGAATTTGCCACCTGGTCCACCATCTC
TCCCAATCATTGGTAACTTACATCATTTGAAGAGACCATTGCACAGAACTTTCAAGGG
ATTGTCTGAAAAGTACGGTCACGTTATCTCTTTGTGGTTCGGTTCTAGATTGGTTGTCG
 
TCGTGTCTTCCTTATCTGTCTTCCAAGAATGCTTCACCAAGAACGATATTGTTCTCGCT
GACAGACCAAGATTCTTGAGTGGTAAATATATCTTCTACAACTACACCACTGTCGGTT
CCTCTTCCTACGGTGAACACTGGAGAAACTTAAGAAGAATCACTTCCTTAGATGTCTT
GTCCACCCACAGAATTAACTCTTTCTCTGGTGTTAGAAGAGATGAAACCCAAAGACT
GATCCAAAAGTTGGCCGAAGAATCATCCACTGATTTTGCTGAAATTGAATTGACCTCC
AAGTTTTACGATATGACTTTCAACAACATCATGAGAATGATTTCCGGTAAGAGATACTA
CGGCGATGACTGTGACATGATGGACATGGAAGAAGCTAAGCAATTCAGGGCCATGGT
TTCCGAATTGCTACAATTGTCTGGTGCTAACAACAAGACCGACTTCATGCCAGTCTTG
AGATTGGTCGATTTCGAAAACTTGGAAAAACGTTTAAAGAAGATCTCCTCTAAGACT
GACACCTTCTTGCGTGGTTTGATCCAAGAACACAGAAATAAGAAGCAACACACCAAC
ACCATGGTTGACCACTTGCTGTCTTTGCAAGAATCTCAACCAGAATACTATACTGACC
AAATCATCAAGGGTTTAGCCTTGGGTATGTTGTTGGCTGGTACCGATTCCTCAGCTGTT
ACTTTGGAATGGGCTCTATCTTGTTTGTTGAACCACCCAGAAGCCTTGAAGAAGGCA
AGAGACGAATTAGAAACCCATGTCGGTCAAGATCGTTTGTTGGAAGAATCCGATTTG
ACAAAATTGCCATACTTGAAGAACATTATCTACGAAACTTTGAGATTGTACACTCCAG
CTCCATTATTGTTGCCTCACTCTTCTTCTGACGAGTGTATCATCGGTGGTTTCAAGGTT
CCAAGAGAAACTATTATTTTGATTAACGCTTGGTCTATTCACCGTGACCCACAAATCTG
GTCTGAAGCTACTTCTTTCAAGCCAGAAAGATTTGAAAAGGAAGGTGAATTGGACAA
ATTAATTGCTTTCGGTCTGGGTAGAAGAGCTTGTCCAGGTGAAGCTTTGGCCTTGAGA
GGTATCTCTTTGACCTTGGGTCTATTGATTCAATGTTTCGAATGGAAGTTGGTTGGTGA
CAAGGAAATTGACATGCGTGAAGAATCTGGTTTCACTTTATCCAGATTAATTCCATTG
AAGGCTATGTGTAAGGCTCGTCCAGTTGCCAACAAATTAGTTAACACTCAAGCTGTCT
CTTTGGGTGATTGA
>SEQ ID NO: 35 (GgVR) 
ATGGCTGAAGGTAAGGGTAGAGTCTGTGTCACCGGTGGTACTGGTTTCCTAGGTT
CATGGTTAATCAAGCGTTTATTGGAAGACGGTTACGGCGTCAACACTACCATCAGATC
CGACCCAGAAAGAAAGAGAGACATTTCTTTCTTGACTAACTTGCCAGGTGCCTCCGA
AAGATTAAAAATCTTCAATGCTGACTTGTCCGACCCAGAATCCTTCGGACCAGCTATT
GAAGGTTGTGTTGGTGTCTTCCACACTGCTTCCCCAATTGACTTCGCCGTCAGTGAAC
CAGAAGAAATTGTCACCAAGCGTACTGTTGATGGTACTTTGGGTATCTTGAAGGCTTG
CAAGAACTCCAAGACTGTTAAGAGAGTTGTCTACACCTCTTCTGGTGCTGCTGTCTCT
TGGGGTGGTACCGAAAAGGATGTCTTGGACGAATCTGACTGGTCCGATGTGGACATG
TTGAGATCTGTCAAGCCATACTCTTGGTCTTACGCTGTTTCCAAGACCATGACTGAAA
AGGCCGTCTTGGAATTCGGTGAACAACACGGTTTGGATGTTGTTACATTGATTTTGCC
 
ATTCATTGTCGGTAGATTTATCTGTCCAAAGATCCCTGACTCTGTTGAGAAAGCTTTGG
TCCTCGTTTTGGGTAAGAGAGAACAAATCGGTGTTACCAGATTCCACATGGTTCATGT
TGAAGACGTTGCTAGAGCTTACATCTTTTTGCTTGAACACCCAAACCCAAAGGGGAG
ATACAACTGTTCTCCATTTATTGTTCCAATCGAAGAAGTTTCTGAATTGTTGTCTGCCA
AATACCCAGAATACCAAATTCCAACCGTTGAAGAATTAAAAGAAATCAAGGGTGCTA
AGTTGCCTGATTTGAAGTCTAAAAAGTTAGTTGATGCTGGTTTCGAATTCAAGTACAC
TATTGAAGATATGTTCGACGATGCCATCCAATGTTGTAAGGAAAAGGGTTATTTGTGA
>SEQ ID NO: 36 (GgPT) 
ATGTCTTCTCTATTTGGCTCTAACGTTTTCGAATCCATTTCCACTTACAGATCTTAC
GCTCCAAAGGCTTCTCAACACAAGAGAAAGATCCAAAAGGAATACAACTTCTTAAGA
TCCAGACAACCATCTTTGAAGCACATTTACAAGGGTATCGAAGGTGGTTCCACTCAA
CAAGAATGTAACAGAAAGTACGTCGTTAAGACCGTTCCAAAACCATCCTTTGAATCT
GACCCAAGATCCATCGACCAAAAGGATATTTTGGAATGTATCAAGAACTTTTTGGATG
CCCTTTATATGTTCACCACTCCATACTCGTTCTTTTCCTCCGCTTTGTACATCATTAGTG
TTTCTTTCATCGTTGTTGAAAAGTTGTCTGACATTTCCCCATTGTTCTTCACCGGTGTC
TTGCAAGCTGTGGTTCCAAACTTGTTCGTCCATATCTACATGACAGGTTTGAACCAAT
TGTGTGATGTCGAAATCGACAAGATCAACAAGCCTTACTTGCCATTGGTCTCTGGTAA
AATTTCCTTTGCCACCGGTGTAATTGTCGTTGCTTCTTGTCTCTTCTTGTCCTTGTGGT
TGGGTTGGATCGTCGGTTCTTGGCCTTCTACCTGTGTCGTCATCTCCATAGCCATGATC
TGGACTGCTTACTCTATCAATGTTCCATTATTGAGATGGAAGAAATACCCAGTTTTGGC
TGCTATGTGTATTTTCTTGTCTTTGGCCGTCGTTAACCCAATTGGTTACTTCTTACACAT
GCAAACTTTCGTCTTCAAGCGTCCAGCTTCCTTCTCTAGACCATTGATCTTCGTTATAG
CTTTCAAATCTTTCTTCTCTTTAGCCGTCGCTTTATTCAAGGACGTCCCAGACATTGAA
GGTGATCAAACCTTCGGTGTCCAATCTTTTGTTGCCCGTTTCGGTAAGAAGAGAGTTT
TCTGGATCTGCATCAGTTTGATTGAAATGGCTTACGGTTTCGCTTTGTTGATGGGTGCT
ACTTCTGCTTTCTTATGGTCTAAGATTGTCACTGTTTTGGGTCACGCTGTTTTGGCATC
ATTCGTTTTGTATAGAGCTAAGTCTATTGATTTGAGATCCAAGACTTCCATTACCTCTTT
CTACATGTTGATCTGGAAGCTAATGTACATGGAATACTTCTTGATGCCATTAGTTAGAT
GA
>SEQ ID NO: 37 (GgPTR) 
ATGGACTCTACTGCTGCCTCCAAGATCTTGGTTATTGGTGGTACTGGTTACATGGG
TCGTTTTCTGGTCGAAGCTTCTGCCAAGGCCGGTCATCCTACCTTTGCTTTAGTACGT
CACAGTACTGTTACCAACCCAGACAAGTCCTCTATTATTCACTCTTTCAACACCCTAG
 
GCGTCAATCTATTGTTGGGTGACATTAATGATCATCAATCTTTGGTCAAGGCCATCAAG
CAAGCTGATGTTGTCATCTCTACCGTTAACCACCAATATATTTCTGACCAATACAAGAT
TATTAGCGCTATCAAGGAAGCAGGTAACATCAAACGTTTCTTCCCATCTGAATTCGGT
AACGACGTTGTCAGAACTCACGGTGTCGACGGTGCTAAGGCTTTGTTCGATATCAAG
GCTAAATTCAGAAGAACCATCGAAGCTGAAGGTATCCCTCACACTTACGTCGTTGCC
AACTTCTTGACTCAACACTTCTTGCCAACTAGATCCAGATTATTGGCTATTGCTGCTCC
ATTAGATAAGGTTGTCATATTGGGTGATGGTAACACCAAGGCCACATTCAACACTGAA
GAAGGTGTTGCTACCTTCACTATCAGAGCTGTCGACGACCCAAGAACTTTGAACAAG
ATCTTATACATTAGACCACCAGCTAACACTTTGTCCTACAACGATTTGGTTTCCTTGTG
GGAAAAGAAGACCGGTAACACCTTGGAAAGAGTTTATGTTCCAGAAGAACAAGTTT
TGAGATTGATCCAAGAATCTTCTTACCCATTGAACATGGCTTTGTCCATCTGTCACGCT
GCTTACGTTAAGGGTGACCACACCAACTACGAAATTGAACCATCCTTCGGTGTTGAA
GCCTTGGAATTGTACCCAGATGTCAAGTTCACCACTGTGGATGACTACTTGGAACAA
AACAGAGACTGTACTCCATTCTACTTAAATCAATTGATTCCAATCACTAACGGTACCA
AATTTTGA
>SEQ ID NO: 38 (GgOC) 
ATGGCTTTCACTTACTCCACCAAGAAATTGGCTTTGTTGTCTATTATCACCATCTT
CATCTCCTCTTTCCCAGAACCATCTACCTCTGTTGACTTGGAATCCGGTTTCCTGCAAT
GTTTTTCCAGTGGTTTGGGTAACTCTAATTCCACTGCAGAATTGGTTTTGACCAAGAA
CTCGTCCTCTTACGCCTCCTTGTTGCAATCCTCCATCCGTAACCTAAGATTCATGGGTA
CTTCCGTCCCAAAGCCAACTTTGATTGTCACTCCAAGAGACTTGTTCCATATCCAAAC
CTCCATCAAATGCAGCGTTAAGCAAGGTTTGCAAATTCGTGTCAGATCCGGTGGTCAT
GACTACGAAGGTTTATCCTACGTCTCCAACGATGACGTTCCATTCTTGATTATCGACTT
GACCAACTTGAGATCCATCACTATCGATATCAAGGATGAAACTGCTTGGGTTCAATCT
GGTGCTACCTTGGGTGAATTATATTACGCTATTGCTAAGAAGTCTAACGTTCACGGTTT
CCCAGCTGGTTCTTGTCCAACTGTCGGTGTTGGTGGTCACTTCTCTGGTGGTGGTTTC
GGTACCATTTTCAGAAAGTACGGTTTAGCCGCTGACAACGTCATTGACGCTCAAATGG
TTGATGTTAACGGTAAGATCTTGAAGAACAGAACTTTGATGGGTGAAGATTTGTTTTG
GGCTATCAGAGGTGGTGGTGGTTCTTCCTTCGGTGTTGTTACCGCTTGGAAGGTCAAG
TTGGTCCATGTTCCTCCAAAAGTTACTGTTTTCAACATTCCAAAGACCTTGGACCAAA
ATGCTTCTACTTTATTCCACAAGTGGCAAATTGTTGCTGATAAGTTGCCAGGTGAATTG
TTTTTGCACTCTGTCATGGGTGTCTCTAACGCTAACTCTATTTCTTCTTCCTCAGGTGG
CGGTAGAACCGTCCTAGTCTCTTTCACCGGTCTATACTTGGGTACCGTTGAAAACTTG
TTGCCATTGATGCAAAACAACTTTGCTGAATTAGGTTTGCAACACAACAACTGTACTG
 
AAATGTCTTGGATTCAATCTGTACTTTTCTTTGCCGGCTACGACCCAATCAACGACAG
TTTGGAGGTCTTACTTCAAAGAAACCAAACTTTCGGTTCTTTCAAGGCCAAATCTGAC
TACGTTATGGAACCTATCCCAACCTCTGGTTTGGAAGGTTTATGGAACATGTTGTTGG
AAGAAAATTCTTCTCCAACTTTGATTTTGACTCCATACGGTGGTAGAATGTCCGAAAT
CTCTGAATCTGAAACCCCATTCCCTCACAGAAACGGATCCATCTACGGTATCCAATAC
TTGGTTTACTGGGATTCTTCTGAAGAAACACCAAAGCACATCGCCTGGATGAGAAGA
GTCTACTCTTACATGGTCTTGTACGTTTCCAAGTTCCCAAGAGCTGCTTACTTGAACTA
CAGAGATTTGGATATTGGTGTCAACCGTGGTAACACTTCTTATGACTATGAAGAAGCT
AAGTCCTGGGGTCTCAAATACTTCAAGTGTAACTTCGAAAGATTGGCCAAGGTTAAG
GCTGAAGTTGACCCATCCAACTTCTTCAGACACGAACAATCCATTCCATTGTTGTTTT
GA
>SEQ ID NO: 39 (GgDMT)

ATGGAACACTTTTATATGTCTTTATTGTTACTATTCGTTACATTGGTTTCTTTGAGC
TTATTCTTCTTGATCTTTTACCACAACAAGCACAACATGAACAACAACAACAATTTGC
CTCCAGGTAAGATGGGTTACCCAGTTATCGGTGAATCTTTGGAATTTTTGTCCATGGGT
TGGAAGGGTCACCCTGAAAAGTTCATTTTCGACAGAATGGTTAGATACTCCAGTGAG
TTAATTAAGACTTCCATCTTGGGTGTTCCAACTGTCATCTTCTGTGGTCCAGCTTGTAA
CAAATTCTTGTTCTCTAACGAAAATAAGTTGGTGACTGCTTGGTGGCCAGACTCTGTC
AACAAAATCTTCCCAACCACTTCTAATTCTAAGGAAGAATCCAAGAAGATGAGAAAG
TTGTTGCCACAATTCTTGAAGCCAGAAGCTTTGCAACGTTACGTTGGCATTATGGACA
CTTTAGCTCAAAGACACTTCGCGTCTTTGTGGGAAGAAAAGACCCATGTCACCGTTTA
TCCTTTGGCTAAGAGATACACTTTCATGTTGGCCTGTAGATTGTTCATGTCTGTTGAAG
ATGAAAACCACGTTGCCAAGTTCAGAGAACCATTCCATTTGTTGGCCTCCGGTATCAT
CTCTGTCCCAATTGACTTGCCATGGACTCCATTCAACAGAGGTATCAAGGCTTCAAAC
TTCATCAGAAAAGAATTGTTGAAAATTATCAGACAAAGAAAGGTCGACTTAGCTCAA
GGTGTCGCTTCCCCAACCCAAGATATCTTGTCTCACATGTTGCTCACCTGTGATGACG
AAAACGGTGAATTCATGACTGAATTGAACATCGCTGACAAGATTTTGGGTTTGTTGAT
TGGTGGTCATGACACCGCTTCCGCTGCTTGCACTTTCATTGTCAAGTACCTAGCTGAA
CTTCCTCACATTTACGATAGAGTCTACCAAGAACAAATGGAAATTGCTAACTCTAAGT
CTCCAGGTGAATTGCTCAACTGGGATGATATCAACAAGATGCGTTACTCTTGGAACGT
TGCTTCTGAAGTTATGAGAGTTGCCCCACCATTGCAAGGTGGTTTCCGTGAAGCTATT
AACGATTTCGTCTTTAATGGTTTCTCCATACCAAAGGGTTGGAAGTTGTACTGGTCTG
CCAACTCCACCCACAAGAACCCAGAATACTTCCCAGCTCCAGAAAAGTTTGACCCAA
CCAGATTCGAAGGTAACGGTCCAGCCGCTTACACCTTCGTCCCGTTCGGTGGTGGAC

CAAGAATGTGTCCAGGTAAGGAATACGCTAGATTGGAAATCCTAGTTTTCATGCACAA
CTTGGTCAAACGTTTTAAGTGGGAAATGTTGATTCCAGAAGAAAAGATTGTTGTCGAT
CCATTGCCTATGCCAGCCAACGACCTACCAATCAGATTATACCCACACAACACTTGA

The nucleotide sequences provided above serve as explanatory purposes only. Any codon-optimized nucleic acid sequence obtained based on the amino acid sequences shall fall within the scope of the present disclosure.

Finally, it should be noted that the examples above are merely used to illustrate the technical solutions of the present disclosure rather than limiting the scope thereof. Although the present disclosure has been described in detail with reference to the foregoing examples, those ordinary skilled in the art will appreciate that they may still modify the technical solutions described in the foregoing examples, or make equivalent substitutions to some or all of the technical features thereof. Such modifications or substitutions do not cause the nature of the corresponding technical solutions to depart from the scope of the technical solutions of the examples of the present disclosure.

In the description of the present disclosure, terms "an embodiment", "some embodiments," "an example," "a specific example," or "some examples" or the like mean that a particular feature, structure, material, or characteristic described in the embodiment or example is included in at least one embodiment or example of the present disclosure. The appearances of the phrases throughout this specification are not necessarily referring to the same embodiment or example of the present disclosure. Furthermore, the particular feature, structure, material, or characteristic may be combined in any suitable manner in one or more embodiments or examples. In addition, various embodiments or examples described in the specification and features in various embodiments or examples may be combined by those skilled in the art without contradiction to each other.

Although explanatory embodiments have been shown and described, it would be appreciated by those skilled in the art that the above embodiments cannot be construed to limit the present disclosure, and changes, alternatives, and modifications can be made in the embodiments in the scope of the present disclosure.

## Claims

1. A method for biosynthesizing glabridin, comprising:
providing a biological host;
engineering the biological host to heterologously express enzymes of a 4'-O-methyltransferase, an isoflavone 2'-hydroxylase, an isoflavone reductase, a vestitone reductase, a prenyltransferase, a pterocarpan reductase, an oxidative cyclase, and a demethylase to obtain an engineered biological host; and
culturing the engineered biological host to obtain the glabridin,
wherein the biological host is yeast,
wherein the glabridin is biosynthesized using daidzein as a substrate.

2. The method for biosynthesizing glabridin according to claim 1, wherein the enzymes heterologously expressed in the engineered biological host consist of the 4'-O-methyltransferase with an amino acid sequence of SEQ ID NO: 1 or SEQ ID NO: 2, the isoflavone 2'-hydroxylase with an amino acid sequence of SEQ ID NO: 3 or SEQ ID NO: 4, the isoflavone reductase with an amino acid sequence of SEQ ID NO: 5 or SEQ ID NO: 6, the vestitone reductase with an amino acid sequence of SEQ ID NO: 7 or SEQ ID NO: 8, the prenyltransferase with an amino acid sequence of SEQ ID NO: 16 or SEQ ID NO: 10, the pterocarpan reductase with an amino acid sequence of SEQ ID NO: 11 or SEQ ID NO: 12, the oxidative cyclase with an amino acid sequence of SEQ ID NO: 13, and the demethylase with an amino acid sequence of SEQ ID NO: 14 or SEQ ID NO: 15.

3. The method for biosynthesizing glabridin according to claim 1, wherein the engineered biological host heterologously expresses the 4'-O-methyltransferase with an amino acid sequence of SEQ ID NO: 1, the isoflavone 2'-hydroxylase with an amino acid sequence of SEQ ID NO: 3, the isoflavone reductase with an amino acid sequence of SEQ ID NO: 5, the vestitone reductase with an amino acid sequence of SEQ ID NO: 7, the prenyltransferase with an amino acid sequence of SEQ ID NO: 16, the pterocarpan reductase with an amino acid sequence of SEQ ID NO: 11, the oxidative cyclase with an amino acid sequence of SEQ ID NO: 13, and the demethylase with an amino acid sequence of SEQ ID NO: 14.

4. The method for biosynthesizing glabridin according to claim 1, wherein the engineered biological host heterologously expresses the 4'-O-methyltransferase with an amino acid sequence of SEQ ID NO: 2, the isoflavone 2'-hydroxylase an the amino acid sequence of SEQ ID NO: 4, the isoflavone reductase with an amino acid sequence of SEQ ID NO: 6, the vestitone reductase with an amino acid sequence of SEQ ID NO: 8, the prenyltransferase with an amino acid sequence of SEQ ID NO: 10, the pterocarpan reductase with an amino acid sequence of SEQ ID NO: 12, the oxidative cyclase with an amino acid sequence of SEQ ID NO: 13, and the demethylase with an amino acid sequence of SEQ ID NO: 15.

5. The method for biosynthesizing glabridin according to claim 1, wherein the yeast comprises *Saccharomyces cerevisiae, Yarrowia lipolytica,* and *Pichia pastoris.*
